# EUROPEAN PATENT SPECIFICATION

(11) **EP 2 331 675 B1**
(45) Date of publication and mention of the grant of the patent: **16.05.2018**
(21) Application number: 09814853.9
(22) Date of filing: 17.09.2009
(51) Int. Cl.: C12N 5/00, A61K 35/12, G01N 33/53, A61K 35/54

(54) **MARKERS OF INDUCED PLURIPOTENT STEM CELLS**
MARKER FÜR INDUZIERTE PLURIPOTENTE STAMMZELLEN
MARQUEURS DE CELLULES SOUCHES PLURIPOTENTES INDUITES

(30) Priority: 18.09.2008 SG 200806945; 14.05.2009 US 178204 P; 15.07.2009 US 225675 P
(43) Date of publication of application: 15.06.2011
(73) Proprietor: Agency for Science, Technology and Research, Singapore 138632 (SG)
(72) Inventor: CHOO, Boon Hwa Andre, Singapore 138668 (SG); OH, Kah Weng Steve, Singapore 138668 (SG)
(74) Representative: Clegg, Richard Ian
(86) International application number: PCT/SG2009/000346
(87) International publication number: WO 2010/033084

(56) References cited:
- EP-A1- 1 970 446
- WO-A1-2007/102787
- HENG LIANG TAN ET AL: "mAb 84, a Cytotoxic Antibody that Kills Undifferentiated Human Embryonic Stem Cells via Oncosis", STEM CELLS, ALPHAMED PRESS, DAYTON, OH, US, vol. 27, no. 8, 1 August 2009 (2009-08-01) , pages 1792-1801, XP008150204, ISSN: 1066-5099, DOI: 10.1002/STEM.109 [retrieved on 2009-04-30]
- CHOO A B ET AL: "Selection against undifferentiated human embryonic stem cells by a cytotoxic antibody recognizing podocalyxin-like protein-1", STEM CELLS, ALPHAMED PRESS, DAYTON, OH, US, vol. 26, no. 6, 1 June 2008 (2008-06-01), pages 1454-1463, XP002538481, ISSN: 1066-5099, DOI: 10.1634/STEMCELLS.2007-0576 [retrieved on 2008-03-20]
- W.N. DE VRIES ET AL: "Reprogramming and Differentiation in Mammals: Motifs and Mechanisms", COLD SPRING HARBOR SYMPOSIA ON QUANTITATIVE BIOLOGY, vol. 73, no. 0, 1 January 2008 (2008-01-01), pages 33-38, XP55023986, ISSN: 0091-7451, DOI: 10.1101/sqb.2008.73.016
- YU JUNYING ET AL: "Induced pluripotent stem cell lines derived from human somatic cells", SCIENCE, AMERICAN ASSOCIATION FOR THE ADVANCEMENT OF SCIENCE, US, vol. 318, no. 5858, 21 December 2007 (2007-12-21), pages 1917-1920, XP009105055, ISSN: 1095-9203, DOI: 10.1126/SCIENCE.1151526
- TAKAHASHI, K. ET AL.: 'Induction of Pluripotent Stem Cells from Adult Human Fibroblasts by Defined Factors' CELL, [Online] vol. 131, 2007, pages 861 - 872, XP002555952 Retrieved from the Internet: <URL:http://www.cell.com/supplemental/50092 -8674(07)01471-7> [retrieved on 2009-12-15]

## Description

### Field of the Invention

The present invention relates to the expression of podocalyxin-like protein (PODXL) on the surface of induced pluripotent stem cells and particularly, although not exclusively, to the use of PODXL as a marker of induced pluripotent stem cells.

### Background to the Invention

Embryonic and other Pluripotent Stem Cells have great potential in therapy. Such cells can be used in regenerative medicine to repair tissues which have been damaged by disease or injury. However, the use of embryonic stem cells in medicine is limited due to the significant ethical concerns associated with the use of human embryos. Recently, the Yamanaka Lab² and Thomson Lab³ demonstrated that human fibroblasts can be reprogrammed by the transient overexpression of a small number of genes into induced pluripotent stem cells (IPSCs) which functionally and phenotypically resemble hESCs. Thus, pluripotent stem cells can be obtained without the need for the destruction of embryos.

These IPSCs functionally and phenotypically resemble embryonic stem cells (ESCs), and avoid the need for the destruction of embryos. Some IPSCs, like hESC, express Oct-4 and other cell surface markers, such as Tra-1-60/81 and SSEA-3/4. However, IPSCs are not identical to ESCs, as shown by a slower doubling time¹¹, differences in the global gene-expression patterns^{2,3} and DNA methylation status². It remains unknown whether nuclear reprogramming is complete¹⁰, and thus whether IPSCs follow a similar pathway to hESCs during differentiation.

This important breakthrough raises the possibility that cellular therapies using patient-specific input cells may be a reality in the future. Unlike hESC where there are ethical concerns and possible issues of immune rejection, IPSCs can be generated from a donor, reprogrammed, differentiated to the appropriate cell type and transplanted back into the donor. Despite its potential, the problem of teratoma formation by residual IPSC after differentiation remains and needs to be addressed.

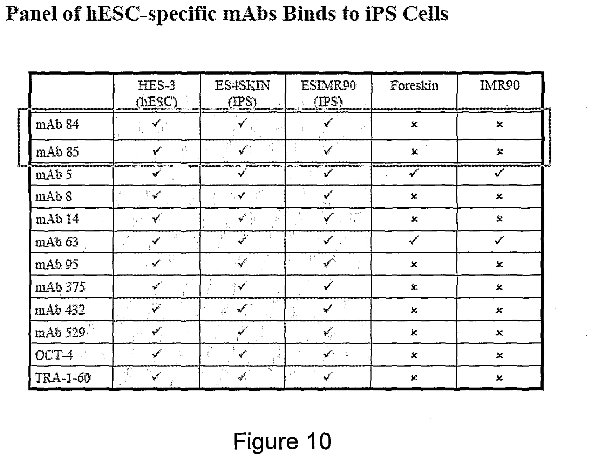

Prior to the publication of reports that IPSCs had been successfully generated from human cells, we described the generation of a panel of monoclonal antibodies (mAbs) against surface antigens on undifferentiated human Embryonic Stem Cells (hESC)¹ in WO 2007/102787,
(also see Figure 10 of the present herein). These mAbs showed strong reactivity against undifferentiated, but not differentiated (embryoid bodies), hESC lines. The mAbs did not cross react with mouse fibroblasts, and showed weak to no reactivity against human embryonal carcinoma cells. Thus these mAbs exhibited very high specificity binding to hESCs, and this binding was lost as the hESCs differentiated. Notably, one antibody (mAb 84), which binds podocalyxin-like protein-1 (PODXL), is cytotoxic to undifferentiated hESC and NCCIT cells in a concentration-dependent, complement-independent manner. mAb 84 induced cell death of undifferentiated, but not differentiated hESC within 30 min of incubation, and immunoprecipitation of the mAb-antigen complex revealed that the antigen is podocalyxin-like protein-1. Importantly, the absence of tumour formation is observed when hESC were treated with mAb 84 prior to transplantation into SCID mice. This earlier data indicates that mAb84 may be useful in eliminating residual hESC from differentiated cell populations for clinical applications.

Although undifferentiated pluripotent stem cells themselves may be used in cell therapy, it is considered to be beneficial to use cells which have started to differentiate, or are differentiated. Methods of encouraging undifferentiated human pluripotent stem cells to differentiate into particular cell lineages are well known in the art. Once this differentiation process has started or proceeded, it is beneficial to remove or destroy undifferentiated pluripotent stem cells which may otherwise form undesirable teratomas.

Thus, it can be seen that it is useful to identify or isolate undifferentiated human pluripotent stem cells (since they can be used themselves in therapy or can be encouraged to differentiate into a particular cell lineage which can be used in therapy). It is also useful to remove or destroy undifferentiated human pluripotent stem cells from a mixture of cells where some of the cells have started to differentiate, or are differentiated, since these differentiated cells are useful in therapy.

### Summary of the Invention

The present invention arises from the discovery that podocalyxin-like protein-1 (PODXL) is a marker of undifferentiated induced pluripotent stem cells (IPSCs).

In the present invention, antibodies that bind to and characterize IPSCs have been discovered. In addition, mAb 84 was further investigated for its ability to kill IPSC.

Accordingly, methods for identifying, isolating, separating, purifying, enriching or removing differentiated or undifferentiated IPSCs are provided. Methods of destroying undifferentiated IPSCs are also provided. Preferably, these methods involve binding of a binding moiety capable of binding PODXL to cells expressing PODXL.

In one aspect of the present disclosure a method of identifying an undifferentiated induced pluripotent stem cell or cells in a sample containing one or a plurality of undifferentiated induced pluripotent stem cells is provided, the method comprising identifying a cell or cells within the sample that express podocalyxin-like protein (PODXL) on their surface.

In some embodiments the sample is contacted with a binding moiety which moiety binds to PODXL and the said undifferentiated induced pluripotent stem cell(s) is identified by virtue of being bound to the binding moiety.

In some embodiments the method further comprises isolating the cell or cells that express PODXL on their surface, wherein the method comprises: (i) contacting the sample with a binding moiety capable of binding PODXL under conditions permitting the binding of the moiety to PODXL expressed on the surface of cells in the sample; and (ii) separating cells bound to the binding moiety from cells not bound to the moiety.

Isolated undifferentiated induced pluripotent stem cell(s) obtained by this method are provided.

In another aspect of the present invention a method of separating undifferentiated induced pluripotent stem cells from induced pluripotent stem cells that have undergone or are undergoing differentiation is provided, wherein the undifferentiated induced pluripotent stem cells and induced pluripotent stem cells that have undergone or are undergoing differentiation are present in a sample, the method comprising: (i) contacting the sample with a binding moiety capable of binding podocalyxin-like protein (PODXL) under conditions permitting the binding of the moiety to PODXL expressed on the surface of cells in the sample; and (ii) separating cells bound to the binding moiety from cells not bound to the moiety.

Isolated undifferentiated induced pluripotent stem cell(s) obtained by this method are provided. Isolated differentiated induced pluripotent stem cell(s) obtained by this method are also provided.

In another aspect of the present disclosure a method of isolating an undifferentiated induced pluripotent stem cell or cells from a sample containing such cells is provided, the method comprising isolating the cell or cells within the sample that express PODXL on their surface.

In some embodiments the sample is contacted with a binding moiety which moiety binds to PODXL and the said undifferentiated induced pluripotent stem cell(s) is isolated from the sample by virtue of being bound to the binding moiety.

Undifferentiated induced pluripotent stem cell(s) isolated by the method are provided.

In a further aspect of the present invention a method of enriching undifferentiated induced pluripotent stem cells from a mixture comprising undifferentiated induced pluripotent stem cells and induced pluripotent stem cells that have undergone or are undergoing differentiation is provided, the method comprising: (i) contacting the mixture with a binding moiety capable of binding podocalyxin-like protein (PODXL) under conditions permitting the binding of the moiety to PODXL expressed on the surface of cells in the mixture; and (ii) separating cells bound to the moiety from cells not bound to the moiety so as to generate a cell population that is enriched in undifferentiated induced pluripotent stem cells.

In another aspect of the present invention a method of enriching induced pluripotent stem cells that have undergone or are undergoing differentiation from a mixture comprising undifferentiated induced pluripotent stem cells and induced pluripotent stem cells that have undergone or are undergoing differentiation is provided, the method comprising: (i) contacting the mixture with a binding moiety capable of binding podocalyxin-like protein (PODXL) under conditions permitting the binding of the moiety to PODXL expressed on the surface of cells in the mixture; and (ii) separating cells not bound to the moiety from cells bound to the moiety so as to generate a cell population that is enriched in induced pluripotent stem cells that have undergone or are undergoing differentiation.

In a further aspect of the present invention a method of preparing a composition containing cells differentiated from undifferentiated induced pluripotent stem cells which composition contains substantially no undifferentiated induced pluripotent stem cells which express PODXL on their surface is provided, the method comprising: (i) providing a population of cells comprising undifferentiated induced pluripotent stem cells and cells differentiated from undifferentiated induced pluripotent stem cells; (ii) contacting the population with a binding moiety capable of binding podocalyxin-like protein (PODXL) under conditions permitting the binding of the moiety to PODXL expressed on the surface of cells in the sample; and (iii) separating cells not bound to the binding moiety from cells bound to the moiety.

In some embodiments the method further comprises mixing the separated cells not bound to the binding moiety with a pharmaceutically acceptable carrier, adjuvant or diluent.

In another aspect of the present invention a method of preparing a composition containing undifferentiated induced pluripotent stem cells which express PODXL on their surface which composition contains substantially no cells differentiated from undifferentiated induced pluripotent stem cells is provided, the method comprising: (i) providing a population of cells comprising undifferentiated induced pluripotent stem cells and cells differentiated from undifferentiated induced pluripotent stem cells; (ii) contacting the population with a binding moiety capable of binding podocalyxin-like protein (PODXL) under conditions permitting the binding of the moiety to PODXL expressed on the surface of cells in the sample; and (iii) separating cells bound to the binding moiety from cells not bound to the moiety.

In some embodiments the method further comprises dissociating the binding moiety from the separated cells bound to the binding moiety. The method may also comprise the step of mixing the separated cells that bound to the binding moiety with a pharmaceutically acceptable carrier, adjuvant or diluent.

In yet another aspect of the present disclosure a method of binding a binding moiety capable of binding PODXL to an undifferentiated induced pluripotent stem cell is provided, the method comprising contacting a sample containing one or more undifferentiated induced pluripotent stem cells with a binding moiety capable of binding PODXL under conditions permitting the binding of the moiety to PODXL expressed on the surface of cells in the sample.

In a further aspect of the present invention a method of destroying an undifferentiated induced pluripotent stem cell or cells in a sample containing such cells is provided, the method comprising destroying the cell or cells in the sample that express PODXL on their surface.

In another aspect of the present invention a method of removing an undifferentiated induced pluripotent stem cell or cells from a sample containing such cells is provided, the method comprising removing from the sample the cell or cells that express PODXL on their surface.

In some embodiments the sample is contacted with a binding moiety which moiety binds to PODXL and the said undifferentiated induced pluripotent stem cell(s) is removed from the sample by virtue of being bound to the binding moiety.

In any of the aspect of the invention described above, in some embodiments the binding moiety is an antibody, and in preferred embodiments is mAb84.

In a further aspect a composition containing cells differentiated from undifferentiated induced pluripotent stem cells is provided, which composition contains substantially no undifferentiated induced pluripotent stem cells which express PODXL on their surface.

In another aspect a composition containing undifferentiated induced pluripotent stem cells is provided, which composition contains substantially no cells differentiated from undifferentiated induced pluripotent stem cells. The undifferentiated induced pluripotent stem cells preferably express PODXL on their surface.

In a further aspect a method of treating a patient in need of cell therapy is provided, the method comprising administering to the patient one or more of: (i) an isolated undifferentiated induced pluripotent stem cell or cells obtained by a method of the present invention; (ii) an isolated differentiated induced pluripotent stem cell or cells obtained by a method of the present invention; or (iii) a composition according to the present invention.

In a further aspect there is provided the use of one or more of: (i) an isolated undifferentiated induced pluripotent stem cell or cells obtained by a method of the present invention; (ii) an isolated differentiated induced pluripotent stem cell or cells obtained by a method of the present invention; or (iii) a composition according to the present invention, in the manufacture of a medicament for treating a patient in need of cell therapy.

In another aspect an isolated undifferentiated induced pluripotent stem cell or cells obtained by a method of the present invention; or an isolated differentiated induced pluripotent stem cell or cells obtained by a method of the present invention; or a composition according to the present invention, is provided for use in treating a patient in need of cell therapy.

In yet a further aspect a complex is provided, the complex comprising an undifferentiated induced pluripotent stem cell expressing podocalyxin-like protein (PODXL) on its surface and a binding moiety capable of binding PODXL which is bound to PODXL expressed on the surface of the undifferentiated induced pluripotent stem cell. The complex is preferably formed by non-covalent and non-ionic interaction of the binding moiety and PODXL. The complex may be provided in isolated form, or as part of a mixture or sample, e.g. part of a culture of cells.

In a further aspect a kit of parts is provided comprising a binding moiety which binds PODXL and a further agent that detects another induced pluripotent stem cell marker.

In a further aspect a method is provided for generating and identifying induced pluripotent stem cells, the method comprising:
(i) inducing non-pluripotent donor cells into a pluripotent state to generate induced pluripotent stem cells expressing PODXL on their surface;
(ii) contacting the induced pluripotent stem cells with a binding moiety capable of binding PODXL under conditions permitting the binding of the moiety to PODXL expressed on the surface of the induced pluripotent stem cells; and
(iii) identifying cells bound by the binding moiety.

In another aspect of the present invention, the use of mAb84 in the destruction of undifferentiated induced pluripotent stem cells is provided. Accordingly, a method of destroying an undifferentiated induced pluripotent stem cell or cells in a sample containing such cells is provided, the method comprising contacting cells in the sample with mAb84 to allow mAb84 to bind certain cells. The method may further comprise culturing or incubating the sample for sufficient time to allow for destruction of cells bound to mAb84.

In yet a further aspect a complex is provided, the complex comprising an undifferentiated induced pluripotent stem cell bound to mAb84. The complex is preferably formed by non-covalent and non-ionic interaction of the binding moiety and PODXL. The complex may be provided in isolated form, or as part of a mixture or sample, e.g. part of a culture of cells.

Further aspects of the present invention are set out below.

In one aspect of the invention, there is provided a method of identifying an undifferentiated induced pluripotent stem cells in a sample which may contain such cells, the method comprising identifying the cell or cells within the sample that express podocalyxin-like protein (PODXL) on their surface.

In another aspect of the invention, there is provided a method of isolating an undifferentiated induced pluripotent stem cells from a sample containing such cells, the method comprising isolating the cell or cells within the sample that express PODXL on their surface.

In another aspect of the invention, there is provided a method of removing an undifferentiated induced pluripotent stem cells from a sample containing such cells, the method comprising removing from the sample the cell or cells that express PODXL on their surface.

In another aspect of the invention, there is provided a method of destroying an undifferentiated induced pluripotent stem cells in a sample containing such cells, the method comprising destroying the cell or cells in the sample that express PODXL on their surface.

In another aspect of the invention, there is provided an undifferentiated induced pluripotent stem cell isolated by a method of isolating an undifferentiated induced pluripotent stem cells from a sample containing such cells, the method comprising isolating the cell or cells within the sample that express PODXL on their surface.

In another aspect there is provided an isolated undifferentiated induced pluripotent stem cell which expresses PODXL on its surface.

In another aspect there is provided a composition containing cells differentiated from undifferentiated induced pluripotent stem cells which composition contains substantially no undifferentiated induced pluripotent stem cells which express PODXL on their surface.

In another aspect there is provided a method of treating a patient in need of cell therapy, the method comprising administering to the patient an undifferentiated induced pluripotent stem cell isolated by a method of isolating an undifferentiated induced pluripotent stem cells from a sample containing such cells or an isolated undifferentiated induced pluripotent stem cell which expresses PODXL on its surface or a composition containing cells differentiated from undifferentiated induced pluripotent stem cells which composition contains substantially no undifferentiated induced pluripotent stem cells which express PODXL on their surface.

In another aspect there is provided the use of a cell according to the above aspects of the invention or a composition according to the above aspects of the invention in the manufacture of a medicament for treating a patient in need of cell therapy.

In another aspect there is provided a method according to the above aspects of the invention wherein the sample is contacted with a binding moiety which moiety binds to PODXL and the said induced pluripotent stem cells is identified in or isolated from or removed from the sample by virtue of being bound by to the binding moiety. Preferably, the binding moiety is an antibody.

In another aspect there is provided a kit of parts comprising a moiety which binds PODXL and a further agent that detects another induced pluripotent stem cells marker.

The following numbered paragraphs (paras.) contain statements of broad combinations of the technical features herein disclosed:
1. A method of identifying an undifferentiated induced pluripotent stem cells in a sample which may contain such cells, the method comprising identifying the cell or cells within the sample that express podocalyxin-like protein (PODXL) on their surface.
2. A method of isolating an undifferentiated induced pluripotent stem cells from a sample containing such cells, the method comprising isolating the cell or cells within the sample that express PODXL on their surface.
3. A method of removing an undifferentiated induced pluripotent stem cells from a sample containing such cells, the method comprising removing from the sample the cell or cells that express PODXL on their surface.
4. A method of destroying an undifferentiated induced pluripotent stem cells in a sample containing such cells, the method comprising destroying the cell or cells in the sample that express PODXL on their surface.
5. An undifferentiated induced pluripotent stem cell isolated by the method of paragraph 2.
6. An isolated undifferentiated induced pluripotent stem cell which expresses PODXL on its surface.
7. A composition containing cells differentiated from undifferentiated induced pluripotent stem cells which composition contains substantially no undifferentiated induced pluripotent stem cells which express PODXL on their surface.
8. A method of treating a patient in need of cell therapy, the method comprising administering to the patient a cell according to paragraph 5 or 6 or a composition according to paragraph 7.
9. Use of a cell according to paragraph 5 or 6 or a composition according to paragraph 7 in the manufacture of a medicament for treating a patient in need of cell therapy.
10. A method according to any one of paragraphs 1 to 3 wherein the sample is contacted with a binding moiety which moiety binds to PODXL and the said induced pluripotent stem cells is identified in or isolated from or removed from the sample by virtue of being bound by to the binding moiety.
11. A method according to paragraph 10 wherein the binding moiety is an antibody.
12. A kit of parts comprising a moiety which binds PODXL and a further agent that detects another induced pluripotent stem cells marker.

### Description of Preferred Embodiments

### Methods

Methods according to the present invention preferably involve binding of a PODXL binding moiety to cells that express PODXL on their surface.

Methods according to the present disclosure may comprise:
(a) identifying undifferentiated induced pluripotent stem cells;
(b) isolating undifferentiated or differentiated induced pluripotent stem cells;
(c) separating undifferentiated induced pluripotent stem cells from other cells, e.g. from differentiated induced pluripotent stem cells;
(d) enriching undifferentiated or differentiated induced pluripotent stem cells;
(e) preparing a composition of differentiated induced pluripotent stem cells having substantially no undifferentiated induced pluripotent stem cells; or
(f) preparing a composition of undifferentiated induced pluripotent stem cells having substantially no differentiated induced pluripotent stem cells.

Methods according to the present invention may involve the step of contacting a sample with a moiety which is capable of binding to PODXL (a PODXL binding moiety). This may be under conditions suitable to permit binding of the binding moiety to PODXL. Such conditions are well known to those of ordinary skill in the art, for example comprising physiological pH and physiological buffer.

The sample may be contacted with the PODXL binding moiety for a sufficient time to allow the binding moiety to bind to PODXL. Sufficient time may, for example, be 5 minutes, 10 minutes, 30 minutes, 45 minutes, 60 minutes, 90 minutes or longer than 2 hours.

In preferred embodiments, methods according to the present invention comprise contacting the sample with a PODXL binding moiety, allowing the PODXL binding moiety to bind to PODXL on the surface of the cell and determining which cells have the PODXL binding moiety bound thereto.

Methods according to the present invention may comprise the step of identifying cells bound by a PODXL binding moiety. Methods according to the present invention may comprise the step of isolating cells bound by a PODXL binding moiety. Methods according to the present invention may comprise the step of partitioning, removing, separating or purifying cells bound by a PODXL binding moiety. Methods according to the present invention may comprise the step of destroying cells bound by a PODXL binding moiety. Methods according to the present invention may further comprise the step of quantifying cells that have been identified, isolated, separated, partitioned, enriched, bound or removed.

The methods of identifying undifferentiated induced pluripotent stem cells disclosed herein are useful for imaging undifferentiated IPSCs, particularly where the cells are tagged with a detectable label, and for characterizing IPSCs.

Methods involving binding a PODXL binding moiety to PODXL expressed on the surface of IPSCs are useful for research into, and the clinical development of, IPSCs.

A PODXL binding moiety bound to PODXL expressed on the surface of a cell forms a complex of binding moiety and PODXL. The cell expressing the PODXL to which the binding moiety is bound forms part of the complex.

Some methods of the present invention comprise the step of detecting the complexes, e.g. by detecting the presence of the binding moiety or by detecting a detectable label coupled to the binding moiety.

Some methods of the present invention comprise the step of partitioning those complexes from the sample or from other non-complexed cells in the sample, and may further comprise the step of detecting the complexes, e.g. by detecting the presence of the binding moiety or a detectable label coupled to the binding moiety.

Some methods of the present invention comprise the step of contacting the sample with a PODXL binding moiety for sufficient time to allow formation of a complex of binding moiety and PODXL, and removing complexed cells from the sample. Such methods are useful in isolation and/or purification of differentiated and/or undifferentiated induced pluripotent stem cells.

In methods of the present invention the PODXL binding moiety may be immobilised on, e.g. conjugated to, a solid support so that the induced pluripotent stem cells can be bound by affinity binding. Conveniently, the solid support comprises any suitable matrix such as agarose, acrylamide, Sepharose™ and Sephadex™. The solid support may be a solid substrate such as a microtitre plate or chip, or a column.

In some embodiments the binding moiety is magnetically labelled (either directly or indirectly) such that, when bound, the human embryonic stem cell can be separated from the rest of the sample upon provision of a suitable magnetic field. Microbeads used for magnetic cell sorting are often termed MACS colloidal super paramagnetic microbeads. Induced pluripotent stem cells labelled in this way may be sorted by magnetic activated cell sorting (MACS).

Other methods of separating cells which comprise a specific cellular marker are known in the art and include FACS (Fluorescence Activated Cell Sorting) for which the binding moiety is labelled with a fluorescent molecule.

Methods according to the present invention may comprise the step of culturing the undifferentiated or differentiated induced pluripotent stem cells which have been bound, identified, isolated, separated, enriched or removed. The methods may also comprise the step of differentiating undifferentiated induced pluripotent stem cells so obtained.

Methods according to the present invention may be used to provide an enriched or substantially isolated composition of differentiated or undifferentiated induced pluripotent stem cells. Such a composition may be used in various ways, for example it may be used in cell therapy or it may be used as a source of cells which are then encouraged to differentiate into a particular cell lineage which is useful for a particular therapy, or it may be used to investigate (*in vitro* or *in vivo*) the factors which allow for the cell to differentiate into other cells.

Typically, the enriched composition of induced pluripotent embryonic stem cells contains at least 50% of the cells as differentiated or undifferentiated human embryonic stem cells, preferably at least 70% or at least 90% or at least 95%. Preferably, all of the cells in the composition are the said differentiated or undifferentiated induced pluripotent stem cells.

Methods for enriching a population of cells preferably involve increasing the concentration of the cells in the sample or increasing the population of cells (i.e. number of cells of a given type), either absolutely or relative to the number of other cells in the sample.

The invention also provides methods of destroying an undifferentiated induced pluripotent stem cell, the method comprising destroying the cell or cells in the sample that express PODXL on their surface. In some embodiments a sample of cells is contacted with a PODXL binding moiety for sufficient time to allow the moiety to bind to those cells expressing PODXL on their surface. The moiety may comprise a cytotoxic agent capable of destroying the cell to which it has bound. Alternatively, the cell may be destroyed by the binding moiety itself, or because of the attachment of the binding moiety. Alternatively, the cell may be destroyed by the interaction of the binding moiety with a cytotoxic agent. Accordingly, the method may comprise addition of a cytotoxic agent capable of interacting with the binding moiety so as to destroy cells bound to the binding moiety.

In some methods, the PODXL binding moiety mediates cell death by an oncosis mechanism, which is a form of cell death resulting from membrane damage leading to an increase in cell permeability (as evidenced by permeability to dyes such as propidium iodide/trypan blue) and cell shrinkage. Cell death induced by the methods of the invention may be preceded by poration of the cell membrane, blebbing and/or cell clumping. In preferred methods of destroying an undifferentiated IPSC, the moiety capable of binding to PODXL may be mAb84.

Suitable cytotoxic moieties, which can be linked to an antibody or other binding moiety, include radioactive atoms, cytotoxic drugs, cytotoxic proteins and enzyme systems that convert a prodrug into a cytotoxic drug. These are well known in the art.

In a preferred embodiment, the invention includes a method of destroying an undifferentiated induced pluripotent stem cell in a sample containing such cells, the method comprising the steps of contacting the sample with a PODXL binding moiety which is toxic to the said cell, and allowing the binding moiety to bind to the PODXL on the surface of the said cell and allowing the binding moiety to kill the said cell. The binding moiety may be an antibody which itself is cytotoxic to the said cell or may include a further moiety which is toxic to the cell.

In some embodiments the PODXL binding moiety is administered to the sample or cells simultaneously or sequentially with a cytotoxic agent. The PODXL binding moiety may be administered in conjunction with one or more other agents that are capable of binding to other stem cell associated molecules in order to ensure complete removal of residual IPSCs.

Methods for destroying undifferentiated IPSCs may be used to remove undifferentiated cells from a population of IPSCs that have been induced to differentiate. Methods for destroying undifferentiated IPSCs may be used prior to transplantation of tissues or organs to eliminate residual IPSCs, thus increasing the success and safety of the graft, particularly by reducing the risk of teratoma formation.

The invention provides an isolated undifferentiated induced pluripotent stem cell(s) which expresses PODXL on its surface, as well as an isolated undifferentiated induced pluripotent stem cell which expresses PODXL on its surface which is bound to a PODXL binding moiety.

The invention also provides methods of generating and identifying reprogrammed induced pluripotent stem cells from a sample. This enables the selection of successfully reprogrammed induced pluripotent stem cells from cells which have not been successfully reprogrammed.

Such a method comprises inducing non-pluripotent donor cells into a pluripotent state to generate induced pluripotent stem cells expressing PODXL on their surface, contacting the induced pluripotent stem cells with a binding moiety capable of binding PODXL under conditions permitting the binding of the moiety to PODXL expressed on the surface of the induced pluripotent stem cells; and identifying cells bound by the binding moiety.

Techniques for the induction of non-pluripotent donor cells into a pluripotent state are known in the art. For example, those reported by Takahashi et al ((2007) Induction of Pluripotent Stem Cells from Adult Human Fibroblasts by Defined Factors. Cell 131(5):861-72) and Yu et al ((2007) Induced Pluripotent Stem Cell Lines Derived from Human Somatic Cells. Science 318(5858):1917-20).

Such techniques include transfection of donor cells with stem cell associated genes and/or transcription factors using viral vectors. Such genes and factors may include one or more of Oct-3/4 (Pouf51) Sox2, c-Myc, Kif4, Nonaog and LIN28, as explained below under "Induced Pluripotent Stem Cells".

Donor cells may comprise adult somatic cells, e.g. fibroblasts, as described below under "Induced Pluripotent Stem Cells".

Methods according to the present invention are preferably performed in vitro. The term "in vitro" is intended to encompass experiments with cells in culture whereas the term "in vivo" is intended to encompass experiments with intact multi-cellular organisms.

### Kits

According to some aspects of the present invention a kit of parts is provided. In some embodiments the marker is one or more of Oct4, SSEA-4, Tra-1-60, Tra-1-81 and GCTM-2. The further agent may be any one or more of mAb 5, mAb 8, mAb 14, mAb 63, mAb 84, mAb 85, mAb 95, mAb 375, mAb 432, mAb 529.

In some embodiments, the kit comprises an antibody to PODXL and an antibody to one or more of Oct4, SSEA-4, Tra-1-60, Tra-1-81 and GCTM-2. Typically, the kit may also contain one or more of: reagents for use in immunochemistry; the antibodies immobilised to a solid support; means for labelling the antibodies; means for linking the antibodies to a cytotoxic moiety.

### Sample

Some methods of the present invention involve a sample containing cells. The sample may be any quantity of cells which contains, or is suspected of containing, one or more undifferentiated induced pluripotent stem cells. The sample may be a culture of cells grown *in vitro.* For example, the culture may comprise a suspension of cells or cells cultured in a culture plate or dish.

In preferred embodiments the sample contains undifferentiated induced pluripotent stem cells. In some embodiments the sample contains undifferentiated induced pluripotent stem cells and induced pluripotent stem cells that have undergone differentiation or are undergoing differentiation. Induced pluripotent stem cells that have undergone differentiation or are undergoing differentiation may no longer be pluripotent and preferably do not express PODXL on their surface.

The sample may be one in which undifferentiated induced pluripotent stem cells have been encouraged (or promoted) to differentiate into particular cell lineages and therefore the sample may contain a mixture of undifferentiated and differentiated cells (because differentiation is often not an efficient process). Typically in such a sample the undifferentiated induced pluripotent stem cells constitute a few percent of the total number of cells. Typically, the differentiated cells in the sample may be cardiomyocytes, pancreatic islets, neuronal progenitor cells or mesenchymal stem cells which are derived (by differentiation) from the induced pluripotent stem cells. Removal (or destruction) of the undifferentiated induced pluripotent stem cells from (or in) such a sample will be useful prior to the clinical application of the sample which contains differentiated cells because, potentially, the undifferentiated cells can form undesirable teratomas. Typically, at least 95% of the undifferentiated induced pluripotent stem cells are removed or destroyed. Preferably, all of the said cells are removed or destroyed.

In some embodiments the sample does not contain non-induced pluripotent cells, e.g. embyronic stem cells (ESCs). In some preferred embodiments the sample does not contain human embyronic stem cells or non-induced human pluripotent cells.

The sample may contain undifferentiated IPSCs expressing PODXL on their surface. In some instances, the sample has been derived from somatic cells that have been induced to pluripotency. In other instances, the sample may comprise IPSCs that have been induced to differentiate into other cells. The sample may contain other non-pluripotent cells, e.g. feeder cells or fibroblasts.

### Podocalyxin-like protein (PODXL)

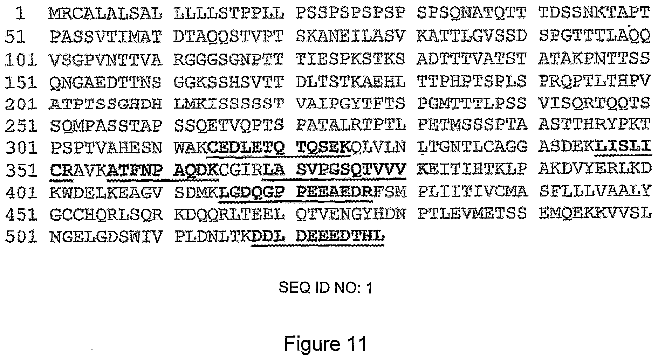

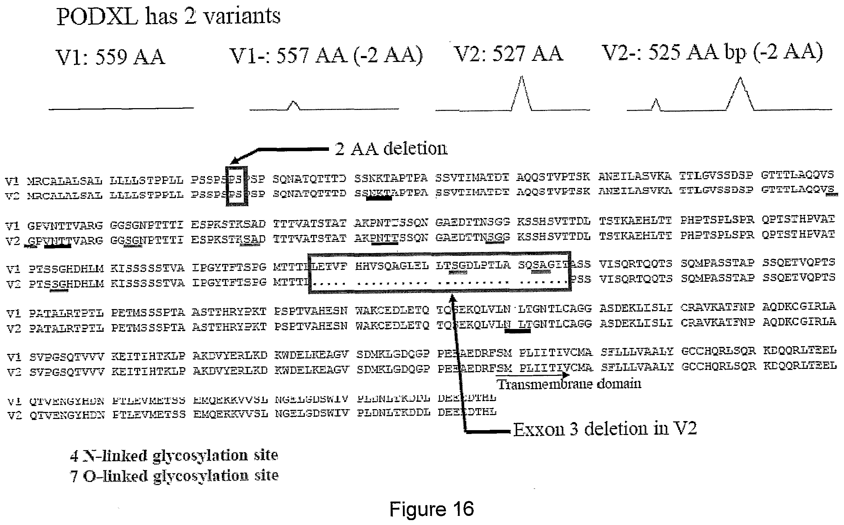

The amino acid sequence of Podocalyxin-like protein 1 precursor herein referred to as Podocalyxin-like protein (PODXL) is given in Figure 11 (SEQ ID NO: 1) and is also found in Accession No. 000592 of the NCBI protein sequence database accessible through EntrezPubMed (see also Kershaw et al (1997) J. Biol. Chem. 272, 15708-15714). It is also called PCLPI and PODXL. For convenience, it will be called PODXL hereafter. PODXL may have the precise sequence given in Figure 11, or it may be a naturally occurring variant thereof (e.g. see Figure 16). For example, according to 000592, R is a variant for the T at residue 62, and S is a variant of the L at residue 196.

Mature PODXL is a 528 residue glycosylated cell surface polypeptide, of which residues 1-22 are a signal peptide, and residues 23-528 represent the mature protein. Residues 23-431 are believed to be the extracellular portion of the protein and residues 432-452 are the transmembrane region. Residues 23-304 represent a Ser/Thr rich region. It is preferred if the binding moiety which binds to PODXL binds to the extracellular region of PODXL, for example within the Ser/Thr rich region, or outside of this region.

Podocalyxin-like protein is a member of the sialomucin protein family. PODXL was originally identified as an important component of glomerular podocytes. Podocytes are highly differentiated epithelial cells with interdigitating foot processes covering the outer aspect of the glomerular basement membrane. Other biological activities of PODXL include binding in a membrane protein complex with Na+/H+ exchanger regulatory factor to intracellular cytoskeletal elements, playing a role in hematopoetic cell differentiation, and being expressed in vascular endothelium cells and binding to L-selectin.

PODXL is a heavily glycosylated type-I transmembrane protein belonging to the CD34 family of sialomucins. PODXL was originally described as the major sialoprotein on podocytes of the kidney glomerulus, but was later found to be expressed on vascular endothelial cells and early hematopoietic progenitors. More recently, PODXL has been implicated as an indicator of tumor aggressiveness in breast, liver, and prostate cancers. Human PODXL is located on chromosome 7q32-q33 and encodes for a protein of 528 amino acids. However, because the extracellular domain of PODXL is extensively glycosylated with sialylated O-linked carbohydrates and five potential sites for N-linked glycosylation, the approximate molecular weight of PODXL is 160-165 kDa.

Functionally, PODXL has been reported to have quite diverse roles depending on the cell type. In podocytes, PODXL acts as an anti-adhesion molecule that maintains the filtration slits open between podocyte foot processes by charge repulsion. However in high endothelial venules, PODXL acts as an adhesion molecule binding to L-selectin and mediating the tethering and rolling of lymphocytes.

In hESC, PODXL was identified transcriptionally to be highly expressed in undifferentiated hESC^{5,6}. By expressed sequence tag frequency analysis, the level of PODXL expression was down-regulated by almost 2.5-fold in 7-8 day embyroid bodies and approximately 7 and 12 fold in neuroectoderm-like cells and hepatocyte-like cells respectively⁵. This result was supported by immunohistochemistry of hESC and 8-day EB where staining was significantly reduced in the latter⁷. In a separate study by Wei et al. comparing the transcriptome profile of hESC and mESC, they observed that the expression of PODXL was not detected by MPSS in mESC line E-14 compared to hESC⁸. At the protein level, Schopperle and DeWolf⁹ reported that PODXL underwent post-translational glycosylation changes after the exposure of 2 embryonal carcinoma lines to retinoic acid (reduction in MW from 200 kDa to 170 kDa). The failure of anti-TRA-1-60/81 antibodies to bind to the modified PODXL prompted them to suggest the presence of a Stem Cell PODXL (SC-PODXL) on embryonic stem cells. Taken together, these independent reports of PODXL expression in ESC corresponds closely with our observations of mAb 84 by flow cytometry where binding reactivity was reduced in day 8 embyroid bodies compared to undifferentiated hESC. Concomitantly, the decrease or loss in mAb 84-mediated killing on FGF2-starved hESC and day 22 EB respectively can be attributed to the down-regulation of PODXL expression upon differentiation. Furthermore, the simultaneous decrease in mAb 84 and TRA-1-60 binding to hESC during embryoid body formation may implicate the loss of SC-PODXL during differentiation.

In preferred methods of the invention, the PODXL is human PODXL. Human PODXL may have the amino acid sequence of SEQ ID NO: 1, or the amino acid sequence of GenBank accession number 000592.2 GI:229462740 (SEQ ID NO: 2; Figure 12) or of GenBank accession number AAI43319.1 GI:219520307 (SEQ ID NO: 3; Figure 13). Some embodiments concern PODXL variants such as those disclosed in Figure 16 (SEQ ID NOs: 8 & 9).

In some embodiments, the PODXL protein comprises one of 60%, 70%, 80%, 85%, 90%, 95%, 96%, 97%, 98%, 99% or 100% sequence identity to the amino acid sequence of SEQ ID NO: 1, SEQ ID NO: 2, SEQ ID NO: 3, SEQ ID NO: 8 or SEQ ID NO: 9. In some embodiments, the PODXL protein comprises one of 60%, 70%, 80%, 85%, 90%, 95%, 96%, 97%, 98%, 99% or 100% sequence identity to the amino acid sequence of SEQ ID NO: 1, SEQ ID NO: 2, SEQ ID NO: 3, SEQ ID NO: 8 or SEQ ID NO: 9, excluding amino acids 1-22. Preferred PODXL proteins are those capable of being bound by mAb84.

### PODXL Binding Moieties

Suitable binding moieties include nucleic acid aptamers (e.g. obtained by Systematic Evolution of Ligands by EXponential enrichment (SELEX)), antibodies (including polyclonal and monoclonal antibodies and antibody fragments), ligands, enzymes and other biological molecules.

It will be appreciated that for all embodiments of the invention it is preferred that the PODXL binding moiety is selective. Thus, it is preferred that the PODXL binding moiety selectively binds PODXL, i.e. binds PODXL with a greater affinity than other human proteins. Typically, the anti-PODXL antibody binds substantially no other human proteins in a selective manner.

Conveniently, the binding moiety is an antibody by which term we include a fragment or derivative thereof, or a synthetic antibody or synthetic antibody fragment.

Antibodies which will bind to PODXL are already known. For example, goat IgG specific for human podocalyxin, which polyclonal antisera binds to the extracellular domain, is available from R&D Systems, Inc under catalogue number AF1658. Also, a monoclonal anti-human podocalyxin antibody (mouse IgG2A) is available from R&D Systems, Inc under catalogue number MAB1658. In view of today's techniques in relation to monoclonal antibody technology, antibodies can be prepared to most antigens. The antigen-binding portion may be a part of an antibody (for example a Fab fragment) or a synthetic antibody fragment (for example a single chain Fv fragment [ScFv]). Suitable monoclonal antibodies to selected antigens may be prepared by known techniques, for example those disclosed in "Monoclonal Antibodies: A manual of techniques ", H Zola (CRC Press, 1988) and in "Monoclonal Hybridoma Antibodies: Techniques and Applications ", J G R Hurrell (CRC Press, 1982). Chimaeric antibodies are discussed by Neuberger et al (1988, 8th International Biotechnology Symposium Part 2, 792-799).

Monoclonal antibodies (mAbs) are useful in the methods of the invention and are a homogenous population of antibodies specifically targeting a single epitope on an antigen. Thus, mAbs binding PODXL can potentially be used to purify or eliminate subsets of viable cells exhibiting a repertoire of antigens that characterizes undifferentiated cells or cells differentiating into a specific lineage.

Polyclonal antibodies are useful in the methods of the invention. Monospecific polyclonal antibodies are preferred. Suitable polyclonal antibodies can be prepared using methods well known in the art.

Fragments of antibodies, such as Fab and Fab₂ fragments may also be used as can genetically engineered antibodies and antibody fragments. The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison et al (1984) Proc. Natl. Acad. Sd. USA 81, 6851-6855).

That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sd. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293- 299.

By "ScFv molecules" we mean molecules wherein the V_{H} and V_{L} partner domains are covalently linked, e.g. by a flexible oligopeptide.

Fab, Fv, ScFv and dAb antibody fragments can all be expressed in and secreted from E. coli, thus allowing the facile production of large amounts of the said fragments.

Whole antibodies, and F(ab')₂ fragments are "bivalent". By "bivalent" we mean that the said antibodies and F(ab')₂ fragments have two antigen combining sites. In contrast, Fab, Fv, ScFv and dAb fragments are monovalent, having only one antigen combining site. Synthetic antibodies which bind to PODXL may also be made using phage display technology as is well known in the art.

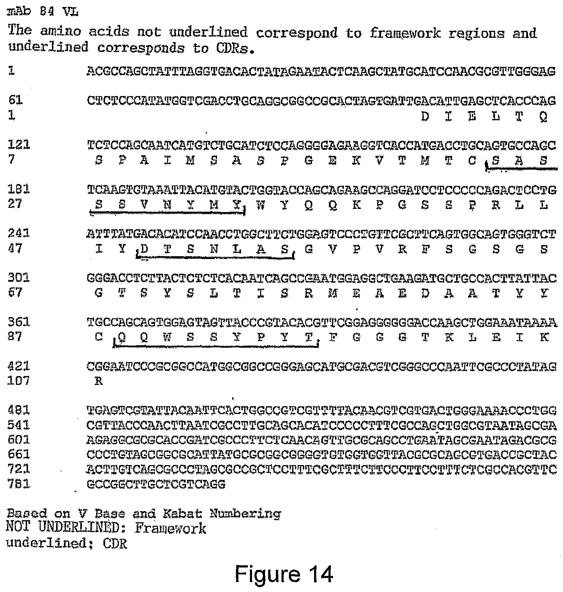

In certain methods, the antibody is mAb84. The amino acid sequence (and encoding polynucleotide sequence) of the V_{H} and V_{L} chains of mAb 84 have been determined as shown in Figures 14 (SEQ ID NO: 4) and 15 (SEQ ID NO: 5).

In this specification, reference to mAb84 includes antibodies having V_{L} and/or V_{H} chains having a high percentage sequence identity to SEQ ID NOs: 4 and 6. Reference to mAb84 includes antibodies that bind PODXL (preferably human PODXL) having a V_{H} chain having at least 70%, more preferably one of at least 75%, 80%, 85%, 90%, 95%, 96%, 97%, 98%, 99% or 100%, sequence identity to SEQ ID NO: 6. Reference to mAb84 includes antibodies that bind PODXL (preferably human PODXL) having a V_{L} chain having at least 70%, more preferably one of at least 75%, 80%, 85%, 90%, 95%, 96%, 97%, 98%, 99% or 100%, sequence identity to SEQ ID NO: 4.

Other mAbs to PODXL and their uses are described in WO 2007/102787.

mAb 84 may be used to eliminate residual undifferentiated induced pluripotent stem cells after differentiation and optionally prior to animal transplantation trials to test the function of their differentiated cells.

mAb 84 (optionally in combination with other mAbs as described in WO 2007/102787 or as set out in Figure 10) may potentially be used prior to transplantation to eliminate residual undifferentiated hESC or undifferentiated induced pluripotent stem cells, thus increasing the success and safety of the graft in regenerative clinical applications.

The PODXL binding moieties of the present application can potentially be used to purify or eliminate subsets of viable cells exhibiting a repertoire of antigens that characterizes undifferentiated cells or cells differentiating into a specific lineage.

A suitable binding moiety is capable of binding to polypeptides having one of at least 65%, at least 70%, at least 75%, at least 80%, at least 85%, at least 90%, at least 95%, at least 99% or 100% sequence identity to one of SEQ ID NOs: 1 to 3, 8 or 9.

In some preferred embodiments the binding moiety is detectably labelled or, at least, capable of detection. For example, the binding moiety may be labelled with a radioactive atom or a coloured molecule or a fluorescent molecule or a molecule which can be readily detected in any other way. Suitable detectable molecules include fluorescent proteins, luciferase, enzyme substrates, and radiolabels. The binding moiety may be directly labelled with a detectable label or it may be indirectly labelled. For example, the binding moiety may be an unlabelled antibody which can be detected by another antibody which is itself labelled. Alternatively, the second antibody may have bound to it biotin and binding of labelled streptavidin to the biotin is used to indirectly label the first antibody.

### Stem Cells

The term "stem cell" generally refers to a cell that on division faces two developmental options: the daughter cells can be identical to the original cell (self-renewal) or they may be the progenitors of more specialised cell types (differentiation). The stem cell is therefore capable of adopting one or other pathway (a further pathway exists in which one of each cell type can be formed). Stem cells are therefore cells which are not terminally differrentiated and are able to produce cells of other types.

As used in this document the term "stem cell" particularly refers to induced pluripotent stem cells.

### Embryonic Stem Cells

Embryonic Stem (ESCs) cells may be isolated from the inner cell mass (ICM) of the blastocyst, which is the stage of embryonic development when implantation occurs.

### Pluripotent Stem Cells

Pluripotent stem cells are true stem cells, with the potential to make any differentiated cell in the body. However, they cannot contribute to making the extraembryonic membranes which are derived from the trophoblast. Several types of pluripotent stem cells have been found.

### Multipotent Stem Cells

Multipotent stem cells are true stem cells but can only differentiate into a limited number of types. For example, the bone marrow contains multipotent stem cells that give rise to all the cells of the blood but not to other types of cells. Multipotent stem cells are found in adult animals. It is thought that every organ in the body contains them where they can replace dead or damaged cells.

Methods of characterising stem cells are known in the art, and include the use of standard assay methods such as clonal assay, flow cytometry, long-term culture and molecular biological techniques e.g. PCR, RT-PCR and Southern blotting.

### Adult Stem Cells

Adult stem cells comprise a wide variety of types including neuronal, skin and the blood forming stem cells which are the active component in bone marrow transplantation.

These latter stem cell types are also the principal feature of umbilical cord-derived stem cells. Adult stem cells can mature both in the laboratory and in the body into functional, more specialised cell types although the exact number of cell types is limited by the type of stem cell chosen.

### Induced Pluripotent Stem Cells

Induced pluripotent stem cells, commonly abbreviated as iPS cells or iPSCs, are a type of pluripotent stem cell artificially derived from a non-pluripotent cell, typically an adult somatic cell, by inserting certain genes. iPS cells are reviewed and discussed in Takahashi, K. & Yamanaka (2006), Yamanaka S, et. al. (2007), Wernig M, et. al. (2007), Maherali N, et. al. (2007), Yu J, et al. (2007) and Takahashi et al., (2007).

iPS cells are typically derived by transfection of certain stem cell-associated genes into non-pluripotent cells, such as adult fibroblasts. Transfection is typically achieved through viral vectors, for example through retroviral reprogramming. Transfected genes include the master transcriptional regulators Oct-3/4 (Pouf51) and Sox2, although it is suggested that other genes enhance the efficiency of induction. After 3-4 weeks, small numbers of transfected cells begin to become morphologically and biochemically similar to pluripotent stem cells, and are typically isolated through morphological selection, doubling time, or through a reporter gene and antibiotic infection.

IPSCs may be induced from somatic cells such as fibroblasts by transfection with one or more transcription factors. In some cases, cells are transformed with Oct3/4, Sox2, c-Myc and Klf4. The cells may be additionally transfected with other genes, including transcription factors and/or marker genes. The genes may be introduced using a transposon system such as the Cre/IoxP recombination system, or using non-integrating vectors in order to produce iPSCs free of exogenous reprogramming genes. Transfection may be achieved using viral vectors, such as a retrovirus. The virus may be an amphotropic virus. Once the cells have been transfected, they may be grown on feeder cells before transfer to an ESC culture medium.

The IPSCs may be derived from rabbit, guinea pig, rat, mouse or other rodent, cat, dog, pig, sheep, goat, cattle, horse, non-human primate or other non-human vertebrate organism. In preferred embodiments the IPSCs are derived from human cells.

iPS cells useful in the invention may be derived from any suitable cell type, including lung, foreskin fibroblasts, skin fibroblasts, keratinocytes, blood progenitor cells, bone marrow cells, hepatocytes, gastric epithelial cells, pancreatic cells, neural stem cells, B lymphocytes, ES derived somatic cells and embryonic fibroblasts. The iPS cells may be derived from human, mouse or other mammals. Preferably, the iPS cells are human. In some cases, the cells are not human dermal fibroblasts. The IPSCs may exhibit similar patterns of gene expression and phenotype to ESCs. In the present invention, the undifferentiated IPSCs express PODXL on their surface.

Like ESCs, future therapeutic applications of differentiated induced pluripotent stem cells carry a risk of teratoma formation by contaminating residual undifferentiated IPSC. Despite this problem, currently there are not many strategies developed to separate these cell populations.

The present invention is particularly concerned with IPSCs expressing PODXL on the cell surface, i.e. IPSCs that have PODXL on their surface which is available for binding to a PODXL binding moiety. The present invention is also particularly concerned with IPSCs derived from human cells.

### Culture of Stem Cells

Any suitable method of culturing stem cells may be used in the methods and compositions described here.

Any suitable container may be used to propagate stem cells according to the methods and compositions described here. Suitable containers include those described in US Patent Publication US2007/0264713 (Terstegge).

Containers may include bioreactors and spinners, for example. A "bioreactor", as the term is used in this document, is a container suitable for the cultivation of eukaryotic cells, for example animal cells or mammalian cells, such as in a large scale. A typical cultivation volume of a regulated bioreactor is between 20 ml and 500 ml.

The bioreactor may comprise a regulated bioreactor, in which one or more conditions may be controlled or monitored, for example, oxygen partial pressure. Devices for measuring and regulating these conditions are known in the art. For example, oxygen electrodes may be used for oxygen partial pressure. The oxygen partial pressure can be regulated via the amount and the composition of the selected gas mixture (e.g., air or a mixture of air and/or oxygen and/or nitrogen and/or carbon dioxide). Suitable devices for measuring and regulating the oxygen partial pressure are described by Bailey, J E. (Bailey, J E., Biochemical Engineering Fundamentals, second edition, McGraw-Hill, Inc. ISBN 0-07-003212-2 Higher Education, (1986)) or Jackson A T. Jackson A T., Verfahrenstechnik in der Biotechnologie, Springer, ISBN 3540561900 (1993)).

Other suitable containers include spinners. Spinners are regulated or unregulated bioreactors, which can be agitated using various agitator mechanisms, such as glass ball agitators, impeller agitators, and other suitable agitators. The cultivation volume of a spinner is typically between 20 ml and 500 ml. Roller bottles are round cell culture flasks made of plastic or glass having a culture area of between 400 and 2000 cm². The cells are cultivated along the entire inner surface of these flasks; the cells are coated with culture medium accomplished by a "rolling" motion, i.e. rotating the bottles about their own individual axis.

Alternatively, culture may be static, i.e. where active agitation of the culture/culture media is not employed. By reducing agitation of the culture, aggregates of cells may be allowed to form. Whilst some agitation may be employed to encourage distribution and flow of the culture media over the cultured cells this may be applied so as not to substantially disrupt aggregate formation. For example, a low rpm agitation, e.g. less than 30rpm or less than 20rpm, may be employed.

### Propagation with Passage

The methods and compositions described here may comprise passaging, or splitting during culture. The methods may involve continuous or continual passage.

By "continual" or "continuous", we mean that our methods enable growth of stem cells in a fashion that enables them to be passaged, e.g., taken off the plates or microcarriers on which they are growing and transferred to other plates, microcarriers or particles, and that this process may be repeated at least once, for example twice, three times, four times, five times, etc. In some cases, this may be repeated any number of times, for example indefinitely or infinitely.

Cells in culture may be dissociated from the substrate or flask, and "split", subcultured or passaged, by dilution into tissue culture medium and replating.

Cells growing on particles may be passaged back onto particle culture. Alternatively, they may be passaged back onto conventional (2D) cultures. Tissue culture cells growing on plates may be passaged onto particle culture.

The term "passage" may generally refer to the process of taking an aliquot of a cell culture, dissociating the cells completely or partially, diluting and inoculating into medium. The passaging may be repeated one or more times. The aliquot may comprise the whole or a portion of the cell culture. The cells of the aliquot may be completely, partially or not confluent. The passaging may comprise at least some of the following sequence of steps: aspiration, rinsing, trypsinization, incubation, dislodging, quenching , re-seeding and aliquoting. The protocol published by the Hedrick Lab, UC San Diego may be used (http://hedricklab.ucsd.edu/Protocol/COSCell.html).

The cells may be dissociated by any suitable means, such as mechanical or enzymatic means known in the art. The cells may be broken up by mechanical dissociation, for example using a cell scraper or pipette. The cells may be dissociated by sieving through a suitable sieve size, such as through 100 micron or 500 micron sieves. The cells may be split by enzymatic dissociation, for example by treatment with collagenase or trypLE harvested. The dissociation may be complete or partial.

The dilution may be of any suitable dilution. The cells in the cell culture may be split at any suitable ratio. For example, the cells may be split at a ratio of 1:2 or more, 1:3 or more, 1:4 or more or 1:5 or more. Thus, stem cells may be passaged for 1 passage or more. For example, stem cells may be passaged for 2, 3, 4, 5, 6, 7, 8, 9, 10, 11, 12, 13, 14, 15, 16, 17, 18, 19, 20, 21, 22, 23, 24, 25 passages or more. Passages may be expressed as generations of cell growth. Our methods and compositions allow stem cells to propagate for 1, 2, 3, 4, 5, 6, 7, 8, 9, 10, 11, 12, 13, 14, 15, 16, 17, 18, 19, 20, 21, 22, 23, 24, 25 generations or more.

Passages may also be expressed as the number of cell doublings. Our methods and compositions allow stem cells to propagate for 1, 2, 3, 4, 5, 6, 7, 8, 9, 10, 11, 12, 13, 14, 15, 16, 17, 18, 19, 20, 21, 22, 23, 24, 25 cell doublings or more.

### Maintenance of Stem Cell Characteristics

The propagated stem cells may retain at least one characteristic of a primate or human stem cell. The stem cells may retain the characteristic after one or more passages. They may do so after a plurality of passages. They may do so after the stated number of passages as described above.

The characteristic may comprise a morphological characteristic, immunohistochemical characteristic, a molecular biological characteristic, etc. The characteristic may comprise a biological activity.

### Stem Cell Characteristics

The stem cells propagated by our methods may display any of the following stem cell characteristics.

Stem cells may display increased expression of Oct4 and/or SSEA-1 and/or TRA-1-60. Stem cells which are self-renewing may display a shortened cell cycle compared to stem cells which are not self-renewing.

Stem cells may display defined morphology. For example, in the two dimensions of a standard microscopic image, human embryonic stem cells display high nuclear/cytopfasmic ratios in the plane of the image, prominent nucleoli, and compact colony formation with poorly discernable cell junctions.

Stem cells may also be characterized by expressed cell markers as described in further detail below.

### Expression of Pluripotency Markers

The biological activity that is retained may comprise expression of one or more pluripotency markers.

Stage-specific embryonic antigens (SSEA) are characteristic of certain embryonic cell types. Antibodies for SSEA markers are available from the Developmental Studies Hybridoma Bank (Bethesda Md.). Other useful markers are detectable using antibodies designated Tra-1-60 and Tra-1-81 (Andrews et al., Cell Lines from Human Germ Cell Tumors, in E. J. Robertson, 1987, supra). Human embryonic stem cells are typically SSEA-1 negative and SSEA-4 positive. hEG cells are typically SSEA-1 positive. Differentiation of primate pluripotent stem cells (pPS) cells in vitro results in the loss of SSEA-4, Tra-1-60, and Tra-1-81 expression and increased expression of SSEA-1. pPS cells can also be characterized by the presence of alkaline phosphatase activity, which can be detected by fixing the cells with 4% paraformaldehyde, and then developing with Vector Red as a substrate, as described by the manufacturer (Vector Laboratories, Burlingame Calif.).

Embryonic stem cells are also typically telomerase positive and OCT-4 positive. Telomerase activity can be determined using TRAP activity assay (Kim et al., Science 266:2011, 1997), using a commercially available kit (TRAPeze.RTM. XK Telomerase Detection Kit, Cat. s7707; Intergen Co., Purchase N.Y.; or TeloTAGGG.TM. Telomerase PCR ELISA plus, Cat. 2,013,89; Roche Diagnostics, Indianapolis). hTERT expression can also be evaluated at the mRNA level by RT-PCR. The LightCycler TeloTAGGG™ hTERT quantification kit (Cat. 3,012,344; Roche Diagnostics) is available commercially for research purposes.

Any one or more of these pluripotency markers, including FOXD3, PODXL, alkaline phosphatase, OCT-4, SSEA-4, TRA-1-60 and Mab84, etc, may be retained by the propagated stem cells.

Detection of markers may be achieved through any means known in the art, for example immunologically. Histochemical staining, flow cytometry (FACS), Western Blot, enzyme-linked immunoassay (ELISA), etc may be used.

Flow immunocytochemistry may be used to detect cell-surface markers. immunohistochemistry (for example, of fixed cells or tissue sections) may be used for intracellular or cell-surface markers. Western blot analysis may be conducted on cellular extracts. Enzyme-linked immunoassay may be used for cellular extracts or products secreted into the medium.

For this purpose, antibodies to the pluripotency markers as available from commercial sources may be used.

Antibodies for the identification of stem cell markers including the Stage-Specific Embryonic Antigens 1 and 4 (SSEA-1 and SSEA-4) and Tumor Rejection Antigen 1-60 and 1-81 (TRA-1-60, TRA-1-81) may be obtained commercially, for example from Chemicon International, Inc (Temecula, CA, USA). The immunological detection of these antigens using monoclonal antibodies has been widely used to characterize pluripotent stem cells (Shamblott M.J. et. al. (1998) PNAS 95: 13726-13731; Schuldiner M. et. al. (2000). PNAS 97: 11307-11312; Thomson J.A. et. al. (1998). Science 282: 1145-1147; Reubinoff B.E. et. al. (2000). Nature Biotechnology 18: 399-404; Henderson J.K. et. al. (2002). Stem Cells 20: 329-337; Pera M. et. al. (2000). J. Cell Science 113: 5-10.).

The expression of tissue-specific gene products can also be detected at the mRNA level by Northern blot analysis, dot-blot hybridization analysis, or by reverse transcriptase initiated polymerase chain reaction (RT-PCR) using sequence-specific primers in standard amplification methods. Sequence data for the particular markers listed in this disclosure can be obtained from public databases such as GenBank (URL www.ncbi.nlm.nih.gov:80/entrez). See U.S. Pat. No. 5,843,780 for further details.

Substantially all of the propagated cells, or a substantial portion of them, may express the marker(s). For example, the percentage of cells that express the marker or markers may be 50% or more, 60% or more, 70% or more, 80% or more, 90% or more, 93% or more, 95% or more, 97% or more, 98% or more, 99% or more, or substantially 100%.

### Cell Viability

The biological activity may comprise cell viability after the stated number of passages. Cell viability may be assayed in various ways, for example by Trypan Blue exclusion.

A protocol for vital staining follows. Place a suitable volume of a cell suspension (20-200 *µ*L) in appropriate tube add an equal volume of 0.4% Trypan blue and gently mix, let stand for 5 minutes at room temperature. Place 10 *µ*l of stained cells in a hemocytometer and count the number of viable (unstained) and dead (stained) cells. Calculate the average number of unstained cells in each quadrant, and multiply by 2 x 10⁴ to find cells/ml. The percentage of viable cells is the number of viable cells divided by the number of dead and viable cells.

The viability of cells may be 50% or more, 60% or more, 70% or more, 80% or more, 90% or more, 93% or more, 95% or more, 97% or more, 98% or more, 99% or more, or substantially 100%.

### Karyotype

The propagated stem cells may retain a normal karyotype during or after propagation. A "normal" karyotype is a karyotype that is identical, similar or substantially similar to a karyotype of a parent stem cell from which the stem cell is derived, or one which varies from it but not in any substantial manner. For example, there should not be any gross anomalies such as translocations, loss of chromosomes, deletions, etc.

Karyotype may be assessed by a number of methods, for example visually under optical microscopy. Karyotypes may be prepared and analyzed as described in McWhir et al. (2006), Hewitt et al. (2007), and Gallimore and Richardson (1973). Cells may also be karyotyped using a standard G-banding technique (available at many clinical diagnostics labs that provide routine karyotyping services, such as the Cytogenetics Lab at Oakland Calif.) and compared to published stem cell karyotypes.
All or a substantial portion of propagated cells may retain a normal karyotype. This proportion may be 50% or more, 60% or more, 70% or more, 80% or more, 90% or more, 93% or more, 95% or more, 97% or more, 98% or more, 99% or more, or substantially 100%.

### Pluripotency

The propagated stem cells may retain the capacity to differentiate into all three cellular lineages, i.e., endoderm, ectoderm and mesoderm. Methods of induction of stem cells to differentiate each of these lineages are known in the art and may be used to assay the capability of the propagated stem cells. All or a substantial portion of propagated cells may retain this ability. This may be 50% or more, 60% or more, 70% or more, 80% or more, 90% or more, 93% or more, 95% or more, 97% or more, 98% or more, 99% or more, or substantially 100% of the propagated stem cells.

The pluripotency of the generated stem cells may be determined by use of suitable assays. Such assays may comprise detecting one or more markers of pluripotency, e.g. SSEA-1 antigen, alkaline phosphatase activity, detection of Oct-4 gene and/or protein expression, by observing the extent of teratoma formation in SCID mice or formation of embryoid bodies. Pluripotency of hESC may be defined by the expression of one or more markers such as Oct-4, SSEA-4, Tra-1-60, Tra-1-81, SOX-2 and GCTM-2.

### Co-Culture and Feeders

Methods may comprise culturing stem cells in the presence or absence of co-culture. The term "co-culture" refers to a mixture of two or more different kinds of cells that are grown together, for example, stromal feeder cells. The two or more different kinds of cells may be grown on the same surfaces, such as particles or cell container surfaces, or on different surfaces. The different kinds of cells may be grown on different particles.

Feeder cells, as the term is used in this document, may mean cells which are used for or required for cultivation of cells of a different type. In the context of stem cell culture, feeder cells have the function of securing the survival, proliferation, and maintenance of cell pluripotency. Cell pluripotency may be achieved by directly co-cultivating the feeder cells. Alternatively, or in addition, the feeder cells may be cultured in a medium to condition it. The conditioned medium may be used to culture the stem cells.

The inner surface of the container such as a culture dish may be coated with a feeder layer of mouse embryonic skin cells that have been treated so they will not divide. The feeder cells release nutrients into the culture medium which are required for ES cell growth. The stem cells growing on particles may therefore be grown in such coated containers.

Arrangements in which feeder cells are absent or not required are also possible. For example, the cells may be grown in medium conditioned by feeder cells or stem cells.

### Media and Feeder Cells

Media for isolating and propagating pluripotent stem cells can have any of several different formulas, as long as the cells obtained have the desired characteristics, and can be propagated further.

Suitable sources are as follows: Dulbecco's modified Eagles medium (DMEM), Gibco#11965-092; Knockout Dulbecco's modified Eagles medium (KO DMEM), Gibco#10829-018; 200 mM L-glutamine, Gibco#15039-027; non-essential amino acid solution, Gibco 11140-050; beta-mercaptoethanol, Sigma#M7522; human recombinant basic fibroblast growth factor (bFGF), Gibco#13256-029. Exemplary serum-containing embryonic stem (ES) medium is made with 80% DMEM (typically KO DMEM), 20% defined fetal bovine serum (FBS) not heat inactivated, 0.1 mM non-essential amino acids, 1 mM L-glutamine, and 0.1 mM beta-mercaptoethanol. The medium is filtered and stored at 4 degrees C for no longer than 2 weeks. Serum-free embryonic stem (ES) medium is made with 80% KO DMEM, 20% serum replacement, 0.1 mM non-essential amino acids, 1 mM L-glutamine, and 0.1 mM beta-mercaptoethanol. An effective serum replacement is Gibco#10828-028. The medium is filtered and stored at 4 degrees C for no longer than 2 weeks. Just before use, human bFGF is added to a final concentration of 4 ng/mL (Bodnar et al., Geron Corp, International Patent Publication WO 99/20741).

The media may comprise Knockout DMEM media (Invitrogen-Gibco, Grand Island, New York), supplemented with 10% serum replacement media (Invitrogen- Gibco, Grand Island, New York), 5ng/ml FGF2 (Invitrogen-Gibco, Grand Island, New York) and 5ng/ml PDGF AB (Peprotech, Rocky Hill, New Jersey).

Feeder cells (where used) may be propagated in mEF medium, containing 90% DMEM (Gibco#11965-092), 10% FBS (Hyclone#30071-03), and 2 mM glutamine. mEFs are propagated in T150 flasks (Coming#430825), splitting the cells 1:2 every other day with trypsin, keeping the cells subconfluent. To prepare the feeder cell layer, cells are irradiated at a dose to inhibit proliferation but permit synthesis of important factors that support human embryonic stem cells (about 4000 rads gamma irradiation). Six-well culture plates (such as Falcon#304) are coated by incubation at 37 degrees C. with 1 mL 0.5% gelatin per well overnight, and plated with 375,000 irradiated mEFs per well. Feeder cell layers are typically used 5 h to 4 days after plating.

Conditions for culturing other stem cells are known, and can be optimized appropriately according to the cell type. Media and culture techniques for particular cell types referred to in the previous section are provided in the references cited.

### Serum Free Media

The methods and compositions described here may include culture of stem cells in a serum-free medium.

The term "serum-free media" may comprise cell culture media which is free of serum proteins, e.g. fetal calf serum. Serum-free media are known in the art, and are described for example in US Patents 5,631,159 and 5,661,034. Serum-free media are commercially available from, for example, Gibco-BRL (Invitrogen).

The serum-free media may be protein free, in that it may lack proteins, hydrolysates, and components of unknown composition. The serum-free media may comprise chemically-defined media in which all components have a known chemical structure. Chemically-defined serum-free media is advantageous as it provides a completely defined system which eliminates variability and allows for improved reproducibility and more consistent performance, and decreases possibility of contamination by adventitious agents.

The serum-free media may comprise Knockout DMEM media (Invitrogen-Gibco, Grand Island, New York).

The serum-free media may be supplemented with one or more components, such as serum replacement media, at a concentration of for example, 5%, 10%, 15%, etc. The serum-free media may be supplemented with 10% serum replacement media from Invitrogen- Gibco (Grand Island, New York).

The serum-free medium in which the dissociated or disaggregated embryonic stem cells are cultured may comprise one or more growth factors. A number of growth factors are known in the art, including FGF2, IGF-2, Noggin, Activin A, TGF beta 1, HRG1 beta, LIF, S1 P, PDGF, BAFF, April, SCF, Flt-3 ligand, Wnt3A and others. The growth factor(s) may be used at any suitable concentration such as between 1 pg/ml to 500ng/ml.

### Media Supplements

Culture media may be supplemented with one or more additives. For example, these may be selected from one or more of: a lipid mixture, Bovine Serum Albumin (e.g. 0.1 % BSA), hydrolysate of soybean protein.

### Sources of Induced Pluripotent Stem Cells

Several methods have now been provided for the isolation of pluripotent stem cells that do not lead to the destruction of an embryo, e.g. by transforming (inducing) adult somatic cells or germ cells. These methods are described herein. The pluripotent stem cells used in the methods according to the invention have not been obtained by a method that involves the destruction of an embryo or otherwise uses a human embryo for industrial or commercial purposes, a method of cloning a human being, or a method of modifying the germ line genetic identity of a human being.
1. Reprogramming by nuclear transfer. This technique involves the transfer of a nucleus from a somatic cell into an oocyte or zygote. In some situations this may lead to the creation of an animal-human hybrid cell. For example, cells may be created by the fusion of a human somatic cell with an animal oocyte or zygote or fusion of a human oocyte or zygote with an animal somatic cell.
2. Reprogramming by fusion with embryonic stem cells. This technique involves the fusion of a somatic cell with an embryonic stem cell. This technique may also lead to the creation of animal-human hybrid cells, as in 1 above.
3. Spontaneous re-programming by culture. This technique involves the generation of pluripotent cells from non-pluripotent cells after long term culture. For example, pluripotent embryonic germ (EG) cells have been generated by long-term culture of primordial germ cells (PGC) (Matsui et al., Derivation of pluripotential embryonic stem cells from murine primordial germ cells in culture. Cell 70, 841-847, 1992). The development of pluripotent stem cells after prolonged culture of bone marrow-derived cells has also been reported (Jiang et al., Pluripotency of mesenchymal stem cells derived from adult marrow. Nature 418, 41-49, 2002). They designated these cells multipotent adult progenitor cells (MAPCs). Shinohara et al also demonstrated that pluripotent stem cells can be generated during the course of culture of germline stem (GS) cells from neonate mouse testes, which they designated multipotent germline stem (mGS) cells (Kanatsu-Shinohara et al., Generation of pluripotent stem cells from neonatal mouse testis. Cell 119, 1001-1012, 2004).
4. Reprogramming by defined factors. For example the generation of iPS cells by the retrovirus-mediated introduction of transcription factors (such as Oct-3/4, Sox2, c-Myc, and KLF4) into mouse embryonic or adult fibroblasts, e.g. as described above. Kaji et al (Virus-free induction of pluripotency and subsequent excision of reprogramming factors. Nature. Online publication 1 March 2009) also describe the non-viral transfection of a single multiprotein expression vector, which comprises the coding sequences of c-Myc, Klf4, Oct4 and Sox2 linked with 2A peptides, that can reprogram both mouse and human fibroblasts. iPS cells produced with this non-viral vector show robust expression of pluripotency markers, indicating a reprogrammed state confirmed functionally by *in vitro* differentiation assays and formation of adult chimaeric mice. They succeeded in establishing reprogrammed human cell lines from embryonic fibroblasts with robust expression of pluripotency markers.
   Methods 1-4 are described and discussed by Shinya Yamanaka in Strategies and New Developments in the Generation of Patient-Specific Pluripotent Stem Cells (Cell Stem Cell 1, July 2007 a2007 Elsevier Inc).
5. Derivation of hESC lines from single blastomeres or biopsied blastomeres. See Klimanskaya I, Chung Y, Becker S, Lu SJ, Lanza R. Human embryonic stem cell lines derived from single blastomeres. Nature 2006; 444:512, Lei et al Xeno-free derivation and culture of human embryonic stem cells: current status, problems and challenges. Cell Research (2007) 17:682-688, Chung Y, Klimanskaya I, Becker S, et al. Embryonic and extraembryonic stem cell lines derived from single mouse blastomeres. Nature. 2006;439:216-219. Klimanskaya I, Chung Y, Becker S, et al. Human embryonic stem cell lines derived from single blastomeres. Nature. 2006;444:481-485. Chung Y, Klimanskaya I, Becker S, et al. Human embryonic stem cell lines generated without embryo destruction. Cell Stem Cell. 2008;2:113-117 and Dusko Ilic et al (Derivation of human embryonic stem cell lines from biopsied blastomeres on human feeders with a minimal exposure to xenomaterials. Stem Cells And Development - paper in pre-publication).
6. hESC lines obtained from arrested embryos which stopped cleavage and failed to develop to morula and blastocysts in vitro. See Zhang X, Stojkovic P, Przyborski S, et al. Derivation of human embryonic stem cells from developing and arrested embryos. Stem Cells 2006; 24:2669-2676 and Lei et al Xeno-free derivation and culture of human embryonic stem cells: current status, problems and challenges. Cell Research (2007) 17:682-688.
7. Parthogenesis (or Parthenogenesis). This technique involves chemical or electrical stimulation of an unfertilised egg so as to cause it to develop into a blastomere from which embryonic stem cells may be derived. For example, see Lin et al. Multilineage potential of homozygous stem cells derived from metaphase II oocytes. Stem Cells. 2003;21(2):152-61 who employed the chemical activation of nonfertilized metaphase II oocytes to produce stem cells.
8. Stem cells of fetal origin. These cells lie between embryonic and adult stem cells in terms of potentiality and may be used to derive pluripotent or multipotent cells. Human umbilical-cord-derived fetal mesenchymal stem cells (UC fMSCs) expressing markers of pluripotency (including Nanog, Oct-4, Sox-2, Rex-1, SSEA-3, SSEA-4, Tra-1-60, and Tra-1-81,minimal evidence of senescence as shown by *β*-galactosidase staining, and the consistent expression of telomerase activity) have been successfully derived by Chris H. Jo et al (Fetal mesenchymal stem cells derived from human umbilical cord sustain primitive characteristics during extensive expansion. Cell Tissue Res (2008) 334:423-433). Winston Costa Pereira et al (Reproducible methodology for the isolation of mesenchymal stem cells from human umbilical cord and its potential for cardiomyocyte generation J Tissue Eng Regen Med 2008; 2: 394-399) isolated a pure population of mesenchymal stem cells from Wharton's jelly of the human umbilical cord. Mesenchymal stem cells derived from Wharton's jelly are also reviewed in Troyer & Weiss (Concise Review: Wharton's Jelly-Derived Cells Are a primitive Stromal Cell Population. Stem Cells 2008:26:591-599). Kim et al (Ex vivo characteristics of human amniotic membrane-derived stem cells. Cloning Stem Cells 2007 Winter;9(4):581-94) succeeded in isolating human amniotic membrane-derived mesenchymal cells from human amniotic membranes. Umbilical cord is a tissue that is normally discarded and stem cells derived from this tissue have tended not to attract moral or ethical objection.

Induced pluripotent stem cells have the advantage that they can be obtained by a method that does not cause the destruction of an embryo, more particularly by a method that does not cause the destruction of a human or mammalian embryo. As such, aspects of the invention may be performed or put into practice by using cells that have not been prepared exclusively by a method which necessarily involves the destruction of human or animal embryos from which those cells may be derived. This optional limitation is specifically intended to take account of Decision G0002/06 of 25 November 2008 of the Enlarged Board of Appeal of the European Patent Office.

### Differentiation of Undifferentiated Cells

IPSCs may be induced to differentiate into a variety of different cell types. For example, the IPSCs may be induced to differentiate into cardiac cells (cardiomyocytes), hepatocytes, neural cells, cartilage (chondrocytes), muscle, fat (adipocytes), bone (osteocytes) or other cells. The IPSCs may be induced to form tissues such as epithelial tissues, mesoderm, endoderm, ectoderm or epidermis.

Methods of differentiating stem cells are known in the art and are described in for example Itskovitz-Eldor (2000) and Graichen et al (2007) and may be used with IPSCs. The cultured stem cells may also be used for the formation of embryoid bodies. Embryoid bodies, and methods for making them, are known in the art. The term "embryoid body" refers to spheroid colonies seen in culture which may be produced by the growth of embryonic stem cells in suspension. Embryoid bodies are of mixed cell types, and the distribution and timing of the appearance of specific cell types corresponds to that observed within the embryo. Embryoid bodies may be generated by plating out embryonic stem cells onto media such as semi-solid media. Methylcellulose media may be used as described in Lim et al, Blood. 1997;90:1291-1299.

Embryonic stem cells may be induced to form embryoid bodies, for example using the methods described in Itskovitz-Eldor (2000). The embryoid bodies contain cells of all three embryonic germ layers (endoderm, ectoderm, mesoderm).

The embryoid bodies may be further induced to differentiate into different lineages for example by exposure to the appropriate induction factor or an environmental change. Graichen et al (2007) describes the formation of cardiomyocytes from human embryonic stem cells by manipulation of the p38MAP kinase pathway. Graichen demonstrates induction of cardiomyocyte formation from stem cells by exposure to a specific inhibitor of p38 MAP kinase such as SB203580 at less than 10 µm.

Differentiated cells may be employed for any suitable purpose, such as regenerative therapy and cell transplantation as known in the art.

### Therapeutic Uses

Differentiated and undifferentiated IPSCs identified and/or isolated by the methods of the present invention have various uses in medicine, for example in cell therapy. Cell therapy may comprise the implantation or transplantation of cells, whether as a population of individual cells, or in the form of a cell aggregate or tissue, and/or regenerative therapy e.g. tissue regeneration, replacement and/or repair. Differentiated and undifferentiated IPSCs may be expanded in *in vitro* culture and directly administered into a patient. They may be used for the repopulation and/or repair of damaged tissue following trauma.

IPSCs may be used directly, or used to generate ectodermal, mesodermal or endodermal progenitor cell populations, for regenerative therapy. Progenitor cells may be made by *ex vivo* expansion or directly administered into a patient. They may also be used for the repopulation and/or repair of damaged tissue following trauma.

Thus, hematopoietic progenitor cells may be used for bone marrow replacement, while cardiac progenitor cells may be used for cardiac failure patients. Skin progenitor cells may be employed for growing skin grafts for patients and endothelial progenitor cells for endothelization of artificial prosthetics such as stents or artificial hearts.

IPSCs may be used as sources of ectodermal, mesodermal or endodermal progenitor cells for the treatment of degenerative diseases such as diabetes, Huntington's disease, Alzheimer's disease and Parkinson's disease. IPSCs may be used as sources of mesodermal or endodermal progenitors for NK or dendritic cells for immunotherapy for cancer.

The methods and compositions described here enable the production of ectodermal, mesodermal or endodermal progenitor cells, which may of course be made to further differentiate using methods known in the art to terminally differentiated cell types.
Thus, any uses of terminally differentiated cells will equally attach to those ectodermal, mesodermal or endodermal progenitor cells (or stem cells) for which they are sources.

IPSCs, ectodermal, mesodermal or endodermal progenitor cells and differentiated cells produced by the methods and compositions described here may be used for, or for the preparation of a pharmaceutical composition for, the treatment of a disease. Such disease may comprise a disease treatable by regenerative therapy, including cardiac failure, bone marrow disease, skin disease, burns, degenerative disease such as diabetes, Alzheimer's disease, Parkinson's disease and cancer.

Stem cells propagated according to the methods and compositions described here (and differentiated cells derived therefrom) may be used for therapy, for example tissue reconstitution or regeneration in an individual patient in need thereof. The cells may be administered in a manner that permits them to graft to the intended tissue site and reconstitute or regenerate the functionally deficient area.

Propagated stem cells or differentiated cells derived therefrom may be used for tissue engineering, such as for the growing of skin grafts. They may be used for the bioengineering of artificial organs or tissues, or for prosthetics, such as stents. Differentiated cells may also be used for tissue reconstitution or regeneration in a human patient in need thereof. The cells are administered in a manner that permits them to graft to the intended tissue site and reconstitute or regenerate the functionally deficient area.

For example, the methods and compositions described here may be used to modulate the differentiation of stem cells. Differentiated cells may be used for tissue engineering, such as for the growing of skin grafts. Modulation of stem cell differentiation may be used for the bioengineering of artificial organs or tissues, or for prosthetics, such as stents.

In another example, neural stem cells are transplanted directly into parenchymal or intrathecal sites of the central nervous system, according to the disease being treated. Grafts are done using single cell suspension or small aggregates at a density of 25,000-500,000 cells per *µ*L (U.S. Pat. No. 5,968,829). The efficacy of neural cell transplants can be assessed in a rat model for acutely injured spinal cord as described by McDonald et al. (Nat. Med. 5:1410, 1999). A successful transplant will show transplant-derived cells present in the lesion 2-5 weeks later, differentiated into astrocytes, oligodendrocytes, and/or neurons, and migrating along the cord from the lesioned end, and an improvement in gate, coordination, and weight-bearing.

Certain neural progenitor cells are designed for treatment of acute or chronic damage to the nervous system. For example, excitotoxicity has been implicated in a variety of conditions including epilepsy, stroke, ischemia, Huntington's disease, Parkinson's disease and Alzheimer's disease. Certain differentiated cells as made according to the methods described here may also be appropriate for treating dysmyelinating disorders, such as Pelizaeus-Merzbacher disease, multiple sclerosis, leukodystrophies, neuritis and neuropathies. Appropriate for these purposes are cell cultures enriched in oligodendrocytes or oligodendrocyte precursors to promote remyelination.

Hepatocytes and hepatocyte precursors prepared using our methods can be assessed in animal models for ability to repair liver damage. One such example is damage caused by intraperitoneal injection of D-galactosamine (Dabeva et al., Am. J. Pathol. 143:1606, 1993). Efficacy of treatment can be determined by immunohistochemical staining for liver cell markers, microscopic determination of whether canalicular structures form in growing tissue, and the ability of the treatment to restore synthesis of liver-specific proteins. Liver cells can be used in therapy by direct administration, or as part of a bioassist device that provides temporary liver function while the subject's liver tissue regenerates itself following fulminant hepatic failure.

Cardiomyocytes may be prepared by inducing differentiation of stem cells by modulation of the MAP kinase pathway for example with SB203580, a specific p38 MAP kinase inhibitor, as described in Graichen et al (2007). The efficacy of such cardiomyocytes may be assessed in animal models for cardiac cryoinjury, which causes 55% of the left ventricular wall tissue to become scar tissue without treatment (Li et al., Ann. Thorac. Surg. 62:654, 1996; Sakai et al., Ann. Thorac. Surg. 8:2074, 1999, Sakai et al., J. Thorac. Cardiovasc. Surg. 118:715, 1999). Successful treatment will reduce the area of the scar, limit scar expansion, and improve heart function as determined by systolic, diastolic, and developed pressure. Cardiac injury can also be modelled using an embolization coil in the distal portion of the left anterior descending artery (Watanabe et al., Cell Transplant. 7:239, 1998), and efficacy of treatment can be evaluated by histology and cardiac function. Cardiomyocyte preparations can be used in therapy to regenerate cardiac muscle and treat insufficient cardiac function (U.S. Pat. No. 5,919,449 and WO 99/03973).

IPSCs can be directed to differentiate into a variety of cell types, and offer the possibility of renewable sources of replacement cells and tissues to treat a range of diseases and disorders. These diseases and disorders include Huntington's disease, Parkinson's disease, Alzheimer's disease, spinal cord injury, bone injury (e.g. fracture), stroke, burns, heart disease, diabetes, osteoarthritis, and rheumatoid arthritis. Diseases and disorders requiring transplantable tissues and organs may be treated, and particularly in cases where it is useful to destroy undifferentiated cells before transplantation, for example to prevent the formation of teratoma.

The invention provides medicaments and pharmaceutical compositions which may comprise undifferentiated induced pluripotent stem cells and differentiated induced pluripotent stem cells isolated by any of the methods of the present invention. The medicaments and pharmaceutical compositions may be provided for use in a method of medical treatment, as described above. Suitable pharmaceutical compositions may further comprise a pharmaceutically acceptable carrier, adjuvant or diluent.

Thus, the invention also provides a pharmaceutical composition and a medicament comprising cells which have been treated to destroy undifferentiated IPSCs, using the methods of the invention.

The ability to identify and select undifferentiated induced pluripotent stem cells through the cell surface expression of PODXL enables compositions and medicaments to be provided which contain undifferentiated induced pluripotent stem cells and substantially no differentiated induced pluripotent stem cells or, alternatively, differentiated induced pluripotent stem cells and substantially no undifferentiated induced pluripotent stem cells. The language "substantially no" includes compositions in which the number of specified cells is less than about 10% of the total number of cells in the composition. More preferably this is less than about 9%, less than about 8%, less than about 7%, less than about 6%, less than about 5%, less than about 4%, less than about 3%, less than about 2%, or less than about 1%. In some embodiments the specified cells may be absent, or present in sufficiently low amounts so as to be beyond the limits of detection, i.e. the composition has substantially zero percent of the specified cells.

Differentiated and undifferentiated induced pluripotent stem cells isolated by a method according to the present invention may be used in a method of medical treatment. A method of medical treatment may comprise administering to an individual in need of treatment a therapeutically effective amount of a said medicament or pharmaceutical composition.

Differentiated and undifferentiated induced pluripotent stem cells obtained through methods and techniques according to this invention may be used to differentiate into another cell type for use in a method of medical treatment. Thus, the differentiated cell type may be derived from, and may be considered as a product of, a stem cell obtained by the methods and techniques described which has subsequently been permitted to differentiate. Pharmaceutical compositions may be provided comprising such differentiated cells, optionally together with a pharmaceutically acceptable carrier, adjuvant or diluent. Such pharmaceutical composition may be useful in a method of medical treatment.

A subject to be treated may be any animal or human. The subject is preferably mammalian, more preferably human. The subject may be male or female. The subject may be a patient. Therapeutic uses may be in human or animals (veterinary use).

Medicaments and pharmaceutical compositions according to aspects of the present invention may be formulated for administration by a number of routes, including but not limited to, parenteral, intravenous, intra-arterial, intramuscular, intratumoural, oral and nasal. The medicaments and compositions may be formulated for injection.

Administration is preferably in a "therapeutically effective amount", this being sufficient to show benefit to the individual. The actual amount administered, and rate and time-course of administration, will depend on the nature and severity of the disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

### Formulating pharmaceutically useful compositions and medicaments

In accordance with the present invention methods are also provided for the production of pharmaceutically useful compositions, which may be based on a cell or cells so identified. In addition to the steps of the methods described herein, such methods of production may further comprise one or more steps selected from:
(a) identifying an undifferentiated or differentiated induced pluripotent stem cell or cells;
(b) isolating and/or obtaining the undifferentiated or differentiated induced pluripotent stem cell or cells;
(c) mixing the undifferentiated or differentiated induced pluripotent stem cell or cells with a pharmaceutically acceptable carrier, adjuvant or diluent.

Step (c) preferably results in formulation/preparation of a pharmaceutical composition or medicament suitable for therapeutic use.

### Sequence Identity

Percentage (%) sequence identity is defined as the percentage of amino acid residues in a candidate sequence that are identical with residues in the given listed sequence (referred to by the SEQ ID NO) after aligning the sequences and introducing gaps if necessary, to achieve the maximum sequence identity, and not considering any conservative substitutions as part of the sequence identity. Sequence identity is preferably calculated over the entire length of the respective sequences.

Where the aligned sequences are of different length, sequence identity of the shorter comparison sequence may be determined over the entire length of the longer given sequence or, where the comparison sequence is longer than the given sequence, sequence identity of the comparison sequence may be determined over the entire length of the shorter given sequence.

Alignment for purposes of determining percent amino acid sequence identity can be achieved in various ways known to a person of skill in the art, for instance, using publicly available computer software such as ClustalW 1.82. T-coffee or Megalign (DNASTAR) software. When using such software, the default parameters, e.g. for gap penalty and extension penalty, are preferably used. The default parameters of ClustalW 1.82 are: Protein Gap Open Penalty = 10.0, Protein Gap Extension Penalty = 0.2, Protein matrix = Gonnet, Protein/DNA ENDGAP = -1, Protein/DNA GAPDIST = 4.

The invention includes the combination of the aspects and preferred features described except where such a combination is clearly impermissible or expressly avoided.

The section headings used herein are for organizational purposes only and are not to be construed as limiting the subject matter described.

Aspects and embodiments of the present invention will now be illustrated, by way of example, with reference to the accompanying figures. Further aspects and embodiments will be apparent to those skilled in the art.

### Brief Description of the Figures

Embodiments and experiments illustrating the principles of the invention will now be discussed with reference to the accompanying figures in which:

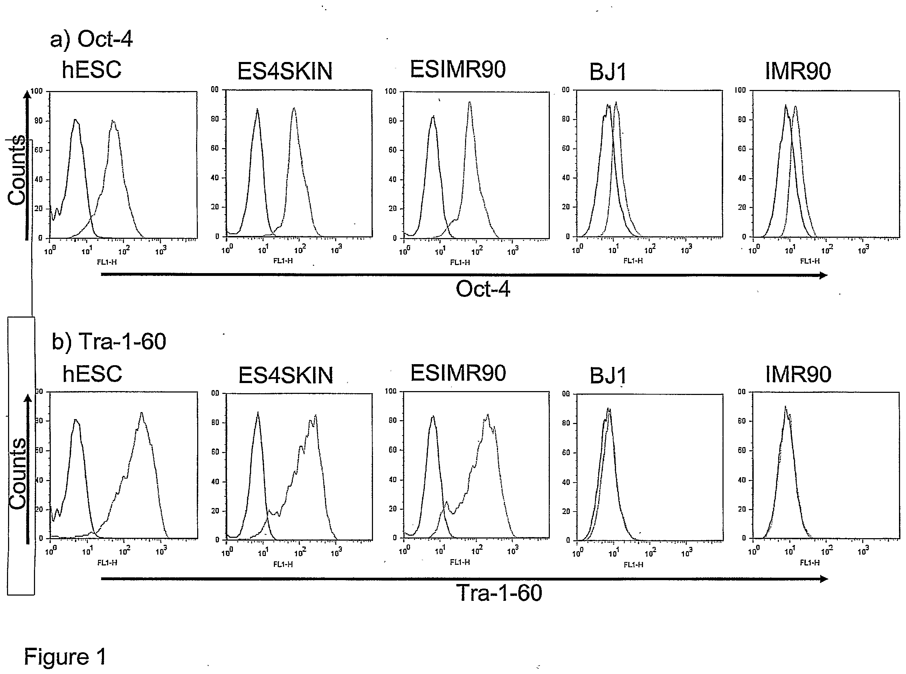

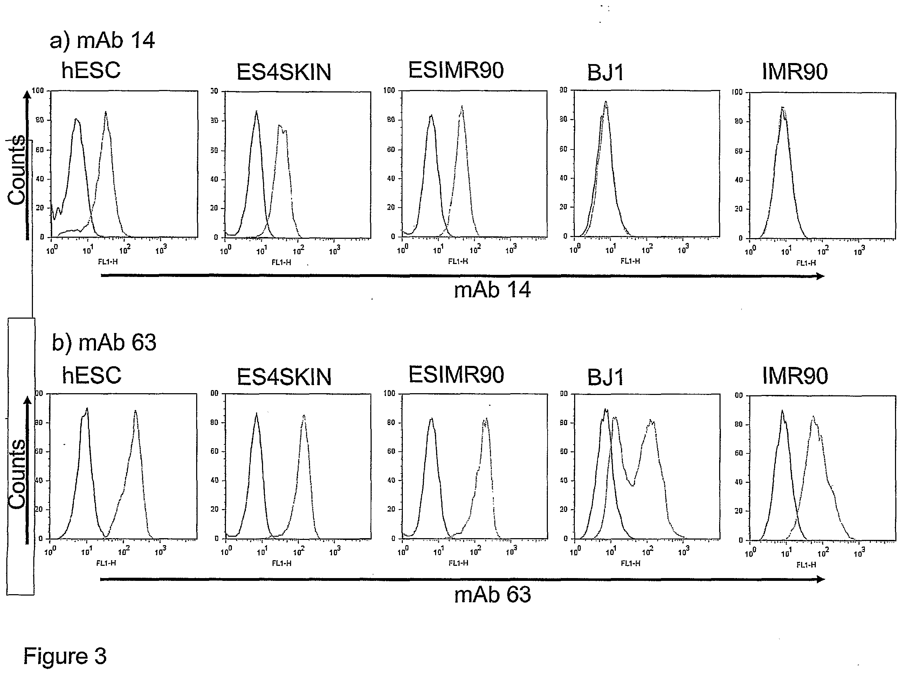

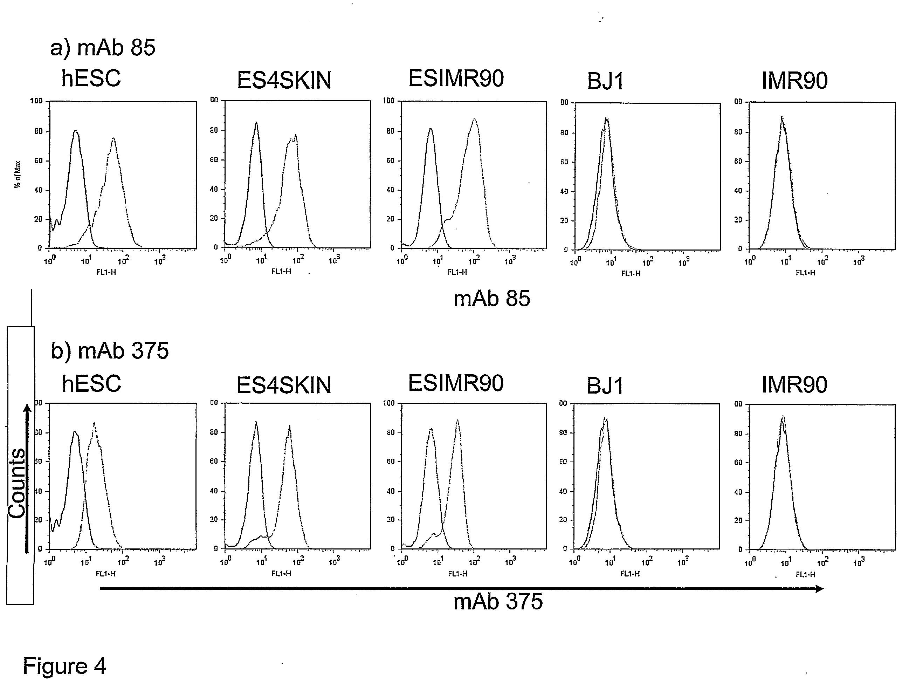

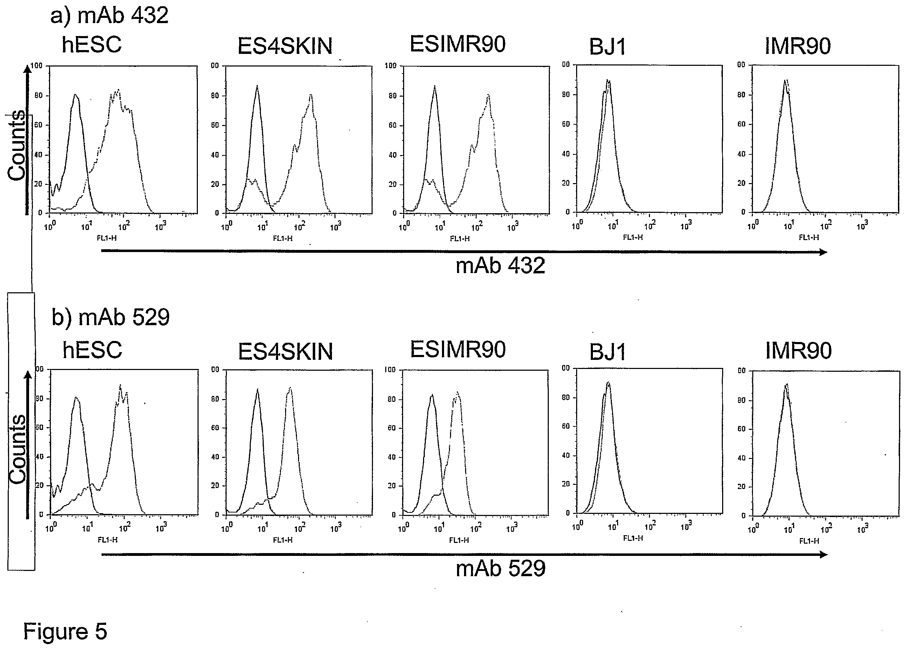

**Figures 1-6** show an analysis of mAb binding to hESC (HES-3), IPSC (ES4SKIN, ESIMR90) and fibroblast cell lines (BJ1, IMR90). Cells were stained with different mAb clones (mAb 5, 8, 14, 63, 84, 85, 329, 432 and 529) raised against cell surface antigens on hESC, mAb to TRA-1-60 or mAb to Oct-4. Antibodies bound to cells were detected with a FITC-conjugated anti-mouse antibody. The red histograms represents staining with the negative control and green histograms represent staining with primary antibodies.

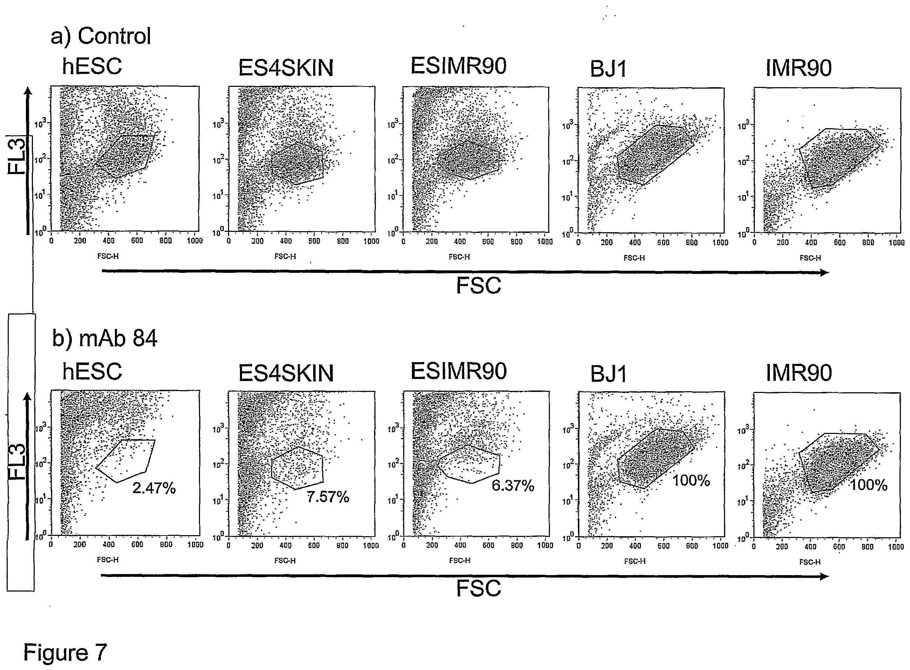

**Figure 7** shows the cytotoxicity of mAb 84 on hESC, IPSC and fibroblast cell lines. (A) Cell were incubated with 5 µg mAb 84 (B) or no antibodies (control, A) at 4°C for 45 min. After which, cells were harvested for analysis by PI exclusion assay on the flow cytometer. Gated region in the scatter plot represents the viable cell population.

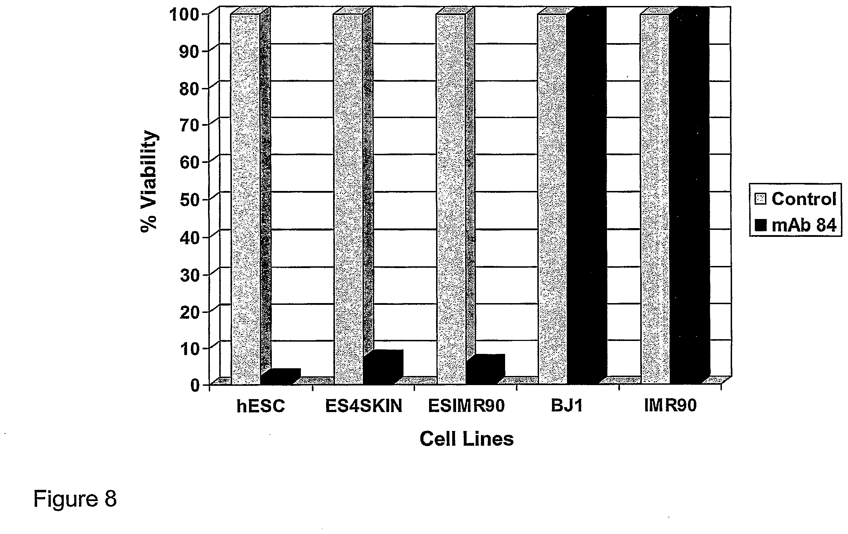

**Figure 8****.** Cytotoxic mAb84 also kills induced pluripotent stem cells. Chart showing summary of results from Figure 7. Percentage viability of cells after incubation with 5 µg mAb 84 (dark blue bars) or no antibodies (light blue bars) at 4°C for 45 min.

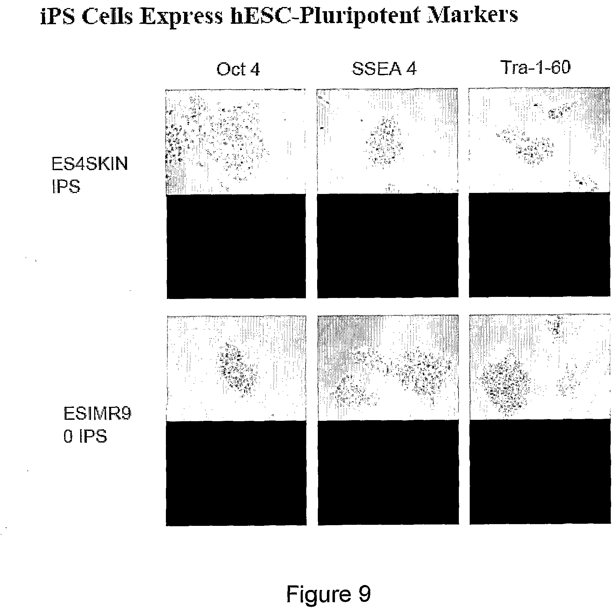

**Figure 9****.** Micrographs showing expression of pluripotency markers Oct4 SSEA 4 and Tra-1-60 by ES4SKIN and ESIMR90 cells.
**Figure 10****.** Table indicating binding (indicated by a tick) of a range of monoclonal antibodies to hESC (HES-3), IPSC (ES4SKIN and ESIMR90), Foreskin and IMR90 cells.
**Figure 11****.** Amino acid sequence of 528 amino acid Podocalyxin-like protein 1 precursor (SEQ ID NO: 1).

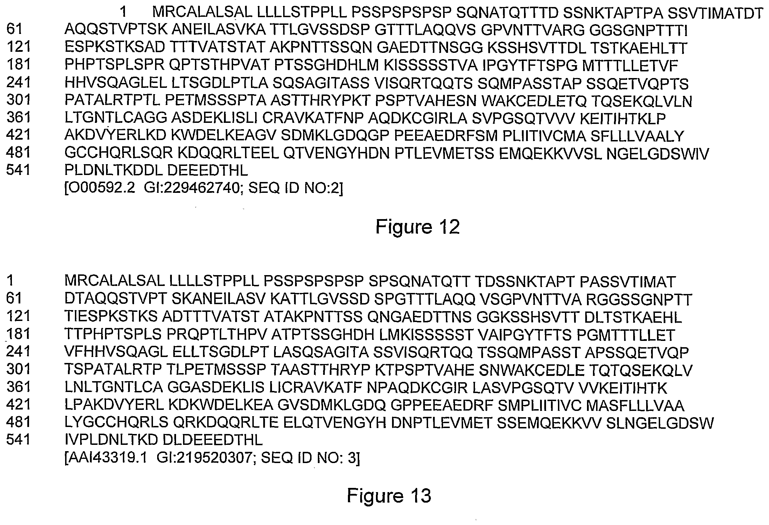

**Figure 12****.** Amino acid sequence of human PODXL GenBank accession number 000592.2 GI:229462740 (SEQ ID NO: 2).
**Figure 13****.** Amino acid sequence of human PODXL GenBank accession number AAI43319.1 GI:219520307 (SEQ ID NO: 3).
**Figure 14****.** Amino acid sequence (and encoding polynucleotide sequence) of the V_{L} chain of mAb 84 (SEQ ID NOs: 4 & 5). Amino acids not underlined correspond to the framework region. The amino acids underlined correspond to the complementarity determining regions (CDRs).

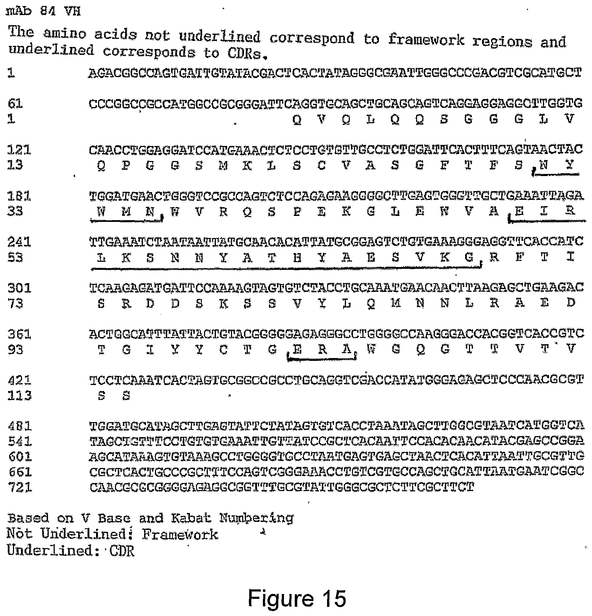

**Figure 15****.** Amino acid sequence (and encoding polynucleotide sequence) of the V_{H} chain of mAb 84 (SEQ ID NO: 6 & 7). The amino acids not underlined correspond to the framework region. The amino acids underlined correspond to the CDRs.
**Figure 16****.** Amino acid sequence of two PODXL variants (SEQ ID NOs: 8 & 9).

### Detailed Description of the Invention

The details of one or more embodiments of the invention are set forth in the accompanying description below including specific details of the best mode contemplated by the inventors for carrying out the invention, by way of example. It will be apparent to one skilled in the art that the present invention may be practiced without limitation to these specific details.

Like hESC, future therapeutic applications of differentiated induced pluripotent stem cells carry a risk of teratoma formation by contaminating residual IPSC. Despite the problem, there are currently not many strategies developed to separate these cell populations. mAbs are a homogenous population of antibodies specifically targeting a single epitope on an antigen. Thus, mAbs of the present application can potentially be used to purify or eliminate subsets of viable cells exhibiting a repertoire of antigens that characterizes undifferentiated cells or cells differentiating into a specific lineage.

mAb 84 may be used to eliminate residual induced pluripotent stem cells after differentiation and prior to animal transplantation trials to test the function of their differentiated cells.

mAb 84 (in combination with other mAbs as described in WO 2007/102787) may potentially be used prior to transplantation to eliminate residual hESC or induced pluripotent stem cells, thus increasing the success and safety of the graft in regenerative medicine applications.

### Reference Example 1

### MATERIALS AND METHODS

### Cell culture

Human embryonic stem cell line HES-3 was obtained from ES Cell International. The cells were cultured at 37°C/5% CO₂ on matrigel-coated organ culture dishes supplemented with conditioned medium from mouse feeders, ΔE-MEF (feeder-free cultures). Medium used for culturing hESC was KNOCKOUT (KO) medium. For feeder-free cultures, hESC were passaged following enzymatic treatment as described previously⁴.

Human induced pluripotent stem cell lines, ES4SKIN and ESIMR90, were obtained from the University of Wisconsin and were cultured as described previously⁴. Human foreskin fibroblast line, BJ1 (CRL-2522) and lung fibroblast line, IMR90 (CCL-186) were purchased from the American Type Culture Collection and cultured according to suppliers instructions.

### Flow cytometry analysis

Cells were harvested as single cell suspensions using trypsin, resuspended at 2 x 10⁵ cells per 10 µl volume in 1% BSA/PBS and incubated for 45 min with each mAb clone (5-10 µg purified mAb in 200 µl 1% BSA/PBS) or TRA-1-60 (2.5 µg in 200 µl 1% BSA/PBS, Chemicon, MAB4360/4381). Cells were then washed with cold 1% BSA/PBS, and further incubated for 15 min with a 1:500 dilution of goat α-mouse antibody FITC-conjugated (DAKO). After incubation, the cells were again washed and resuspended in 1% BSA/PBS and 1.25 mg/ml propidium iodide (PI) for analysis on a FACScan (Becton Dickinson FACS Calibur). All incubations were performed at 4°C unless otherwise indicated. As a negative control, cells were stained with the appropriate isotype control.

For Oct-4 staining, single cell suspensions were fixed, permeabilized (Caltag Laboratories) and incubated with a mouse mAb to Oct-4 (Santa Cruz, sc-5279) at a 1:20 dilution. Cells were then washed with 1% BSA/PBS, and further incubated for 15 min with a 1:500 dilution of goat α-mouse antibody FITC-conjugated (DAKO). After incubation, the cells were again washed and resuspended in 1% BSA/PBS for analysis.

All incubations were performed at room temperature for 15 min. As a negative control, cells were stained with the appropriate isotype control.

### Cytotoxicity assays

Cytotoxicity of mAb 84 on cells was evaluated using PI exclusion assays and flow cytometry. As described above, single cell suspensions at 2 x 10⁵ cells per 10 µl volume in 1 % BSA/PBS was incubated with mAb 84 (5 µg purified mAb in 200 µl 1% BSA/PBS) for 45 min. After which, cells were washed and resuspended in 1% BSA/PBS and 1.25 mg/ml propidium iodide (PI) for analysis by FACS. As a negative control, cells were incubated without a primary antibody. All incubations were performed at 4°C unless otherwise indicated.

### RESULTS

### Reactivity of mAbs with hESC, IPSC and fibroblast cell lines

In the first instance, all the cell lines were tested for the expression of pluripotent markers Oct-4 and Tra-1-60 (Figure 1 a and b respectively). As expected, only hESC and IPSC expressed both markers whilst expression was significantly down-regulated in both the fibroblast cell lines.

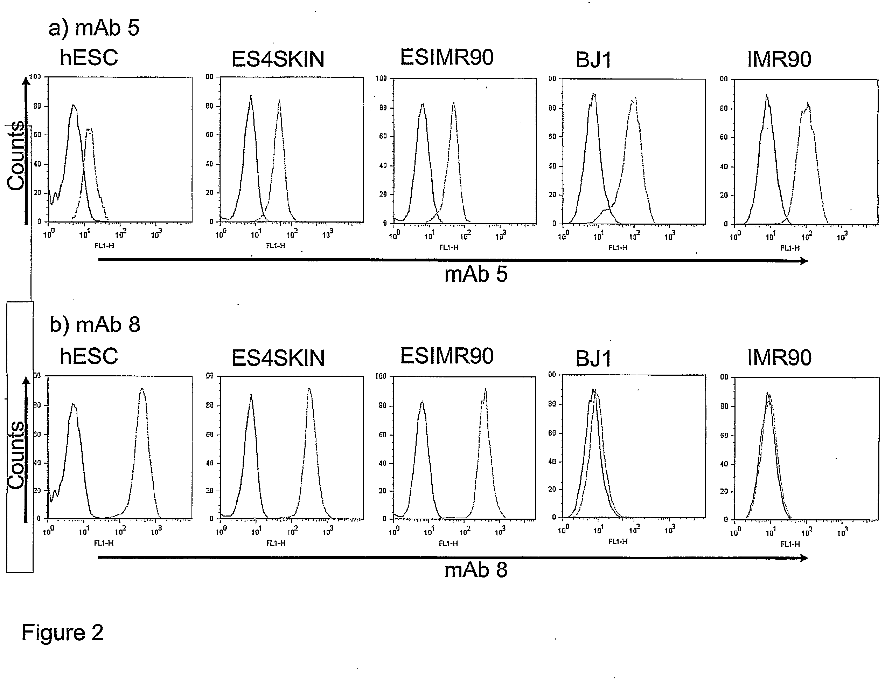

Subsequently, the panel of 9 in-house mAbs to hESC was screened for binding to the 5 cell lines (Figures 2-6). Similar to hESC, all 9 mAbs were found to bind to hESC and IPSC. Since ES4SKIN and ESIMR90 were reprogrammed from primary fibroblast from foreskin and lung respectively, these primary cell lines were incorporated in the screen to identify antibodies that only bind to the "reprogrammed" cell line and not the parental fibroblast line. Based on the screening results, 7 of the mAb clones do not bind to the primary fibroblast. However, reactivity was observed for mAb 5 and mAb 63. This result is consistent with previous observations with hESC¹. This result suggests that the expression of the antigen targets for these 2 mAbs clones may be conserved in all human cells and in particular, for mAb 5, upregulated in terminally differentiated primary fibroblast compared to hESC or IPSC.

### Characterization of the cytotoxic antibody mAb 84

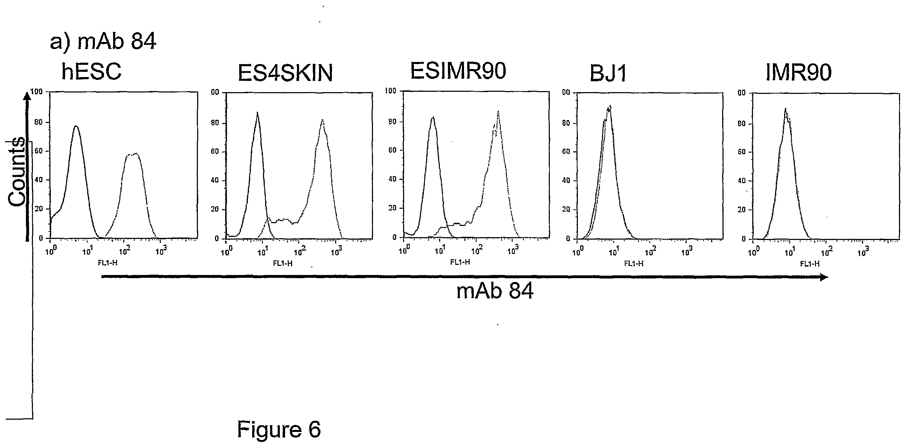

To investigate if mAb 84 kills IPSC similar to hESC, cells were incubated with mAb 84 and cytotoxicity evaluated using PI exclusion assays. It was found that 2.5, 7.6 and 6.4% of hESC, ES4SKIN and ESIMR90 cells respectively remained viable after incubation with mAb 84 (45 min at 4°C) compared to the control (Figure 7 and 8). In contrast, the viability remained at 100% for the BJ1 and IMR90 fibroblast lines. Taking together binding and cytotoxicity data for mAb 84 (Figures 6-9), it can be inferred that mAb 84 binds and kills both hESC and IPSC. This cytotoxic effect of mAb 84 is correlated with the binding of the mAb to the cell lines, as mAb 84 does not exert a cytotoxic effect on cells that do not bind the mAb (BJ1 and IMR90).

### References

1. Choo et al (2008). Selection Against Undifferentiated Human Embryonic Stem Cells By A Cytotoxic Antibody Recognizing Podocalyxin-like Protein-1. Stem Cells 26(6):1454-63.
2. Takahashi et al (2007) Induction of Pluripotent Stem Cells from Adult Human Fibroblasts by Defined Factors. Cell 131(5):861-72.
3. Yu et al (2007) Induced Pluripotent Stem Cell Lines Derived from Human Somatic Cells. Science 318(5858):1917-20.
4. Choo et al (2004) Expansion of pluripotent human embryonic stem cells on human feeders. Biotechnology and .Bioengineering 88:321-331.
5. Brandenberger et al (2004) Transcriptome characterization elucidates signaling networks that control human ES cell growth an differentiation. Nature Biotechnology 22:707-716
6. Cai et al (2006) Assessing self-renewal and differentiation in human embryonic stem cell limes. Stem cells 24:516-530
7. Cai et al (2005) Development of antibodies to human embryonic stem cell antigens. BMC Dev Biol 5:26
8. Wei et al (2005) Transcriptome profiling of human and murine ESCs identifies divergent paths required to maintain the stem cell state. Stem Cells 23:166-185
9. Schopperle et al. (2007) The TRA-1-60 and TRA-1-81 human pluripotent stem cell markers are expressed on podocalyxin in embryonal carcinoma. Stem cells 25:723-730.
10. Yamanaka et al (2009) A Fresh Look at iPS Cells. Cell 137: 13-17
11. Okita et al (2007) Generation of germline-competent induced pluripotent stem cells. Nature 448:313-318.
12. Chung Y, Klimanskaya I, Becker S, et al. Embryonic and extraembryonic stem cell lines derived from single mouse blastomeres. Nature. 2006;439:216-219.
13. Chung Y, Klimanskaya I, Becker S, et al. Human embryonic stem cell lines generated without embryo destruction. Cell Stem Cell. 2008;2:113-117.
14. Jiang, Y., Jahagirdar, B.N., Reinhardt, R.L., Schwartz, R.E., Keene, C.D., Ortiz-Gonzalez, X.R., Reyes, M., Lenvik, T., Lund, T., Blackstad, M., et al. (2002). Pluripotency of mesenchymal stem cells derived from adult marrow. Nature 418, 41-49.
15. Kaji et al. Virus-free induction of pluripotency and subsequent excision of reprogramming factors. Nature. Online publication 1 March 2009.
16. Kanatsu-Shinohara, M., Inoue, K., Lee, J., Yoshimoto, M., Ogonuki, N., Miki, H., Baba, S., Kato, T., Kazuki, Y., Toyokuni, S., et al. (2004). Generation of pluripotent stem cells from neonatal mouse testis. Cell 119, 1001-1012.
17. Kershaw et al (1997) J. Biol. Chem. 272, 15708-15714.
18. Kim et al. Ex vivo characteristics of human amniotic membrane-derived stem cells. Cloning Stem Cells 2007 Winter;9(4):581-94.
19. Lin et al. Multilineage potential of homozygous stem cells derived from metaphase II oocytes. Stem Cells. 2003;21(2):152-61.
20. Lei et al Xeno-free derivation and culture of human embryonic stem cells: current status, problems and challenges. Cell Research (2007) 17:682-688.
21. Maherali N, et. al. Directly reprogrammed fibroblasts show global epigenetic remodeling and widespread tissue contribution. Cell Stem Cell 2007;1:55-70.
22. Matsui, Y., Zsebo, K., and Hogan, B.L. (1992). Derivation of pluripotential embryonic stem cells from murine primordial germ cells in culture. Cell 70, 841-847.
23. Takahashi K, Tanabe K, Ohnuki M, Narita M, Ichisaka T, Tomoda K, Yamanaka S. (2007). Induction of pluripotent stem cells from adult human fibroblasts by defined factors. Cell. 131(5):861-72.
24. Takahashi, K. & Yamanaka, S. Induction of pluripotent stem cells from mouse embryonic and adult fibroblast cultures by defined factors. Cell 2006;126:663-676.
25. Wernig M, et. al. In vitro reprogramming of fibroblasts into a pluripotent ES-cell-like state. Nature 2007;448:318-24.
26. Yamanaka S, et. al. Generation of germline-competent induced pluripotent stem cells. Nature 2007;448:313-7.
27. Yu J, Vodyanik MA, Smuga-Otto K, Antosiewicz-Bourget J, Frane JL, Tian S, Nie J, Jonsdottir GA, Ruotti V, Stewart R, Slukvin II, Thomson JA. Induced pluripotent stem cell lines derived from human somatic cells. Science. 318(5858):1917-20. Epub 2007 Nov 20.
28. Zhang X, Stojkovic P, Przyborski S, et al. Derivation of human embryonic stem cells from developing and arrested embryos. Stem Cells 2006; 24:2669-2676.

## Claims

1. A method of separating undifferentiated induced pluripotent stem cells from induced pluripotent stem cells that have undergone or are undergoing differentiation, wherein the undifferentiated induced pluripotent stem cells and induced pluripotent stem cells that have undergone or are undergoing differentiation are present in a sample, the method comprising:
(i) contacting the sample with a binding moiety capable of binding podocalyxin-like protein (PODXL) under conditions permitting the binding of the moiety to PODXL expressed on the surface of cells in the sample; and
(ii) separating cells bound to the binding moiety from cells not bound to the moiety.

2. A method of enriching undifferentiated induced pluripotent stem cells from a mixture comprising undifferentiated induced pluripotent stem cells and induced pluripotent stem cells that have undergone or are undergoing differentiation, the method comprising:
(i) contacting the mixture with a binding moiety capable of binding podocalyxin-like protein (PODXL) under conditions permitting the binding of the moiety to PODXL expressed on the surface of cells in the mixture; and
(ii) separating cells bound to the moiety from cells not bound to the moiety so as to generate a cell population that is enriched in undifferentiated induced pluripotent stem cells.

3. A method of enriching induced pluripotent stem cells that have undergone or are undergoing differentiation from a mixture comprising undifferentiated induced pluripotent stem cells and induced pluripotent stem cells that have undergone or are undergoing differentiation, the method comprising:
(i) contacting the mixture with a binding moiety capable of binding podocalyxin-like protein (PODXL) under conditions permitting the binding of the moiety to PODXL expressed on the surface of cells in the mixture; and
(ii) separating cells not bound to the moiety from cells bound to the moiety so as to generate a cell population that is enriched in induced pluripotent stem cells that have undergone or are undergoing differentiation.

4. A method of preparing a composition containing cells differentiated from undifferentiated induced pluripotent stem cells which composition contains substantially no undifferentiated induced pluripotent stem cells which express PODXL on their surface, the method comprising:
(i) providing a population of cells comprising undifferentiated induced pluripotent stem cells and cells differentiated from undifferentiated induced pluripotent stem cells;
(ii) contacting the population with a binding moiety capable of binding podocalyxin-like protein (PODXL) under conditions permitting the binding of the moiety to PODXL expressed on the surface of cells in the sample; and
(iii) separating cells not bound to the binding moiety from cells bound to the moiety.

5. The method of claim 4 further comprising mixing the separated cells not bound to the binding moiety with a pharmaceutically acceptable carrier, adjuvant or diluent.

6. A method of preparing a composition containing undifferentiated induced pluripotent stem cells which express PODXL on their surface which composition contains substantially no cells differentiated from undifferentiated induced pluripotent stem cells, the method comprising:
(i) providing a population of cells comprising undifferentiated induced pluripotent stem cells and cells differentiated from undifferentiated induced pluripotent stem cells;
(ii) contacting the population with a binding moiety capable of binding podocalyxin-like protein (PODXL) under conditions permitting the binding of the moiety to PODXL expressed on the surface of cells in the sample; and
(iii) separating cells bound to the binding moiety from cells not bound to the moiety.

7. The method of claim 6 further comprising:
(i) dissociating the binding moiety from the separated cells bound to the binding moiety, and/or
(ii) mixing the separated cells that bound to the binding moiety with a pharmaceutically acceptable carrier, adjuvant or diluent.

8. A method of destroying an undifferentiated induced pluripotent stem cell or cells in a sample containing such cells, the method comprising destroying the cell or cells in the sample that express PODXL on their surface.

9. A method of removing an undifferentiated induced pluripotent stem cell or cells from a sample containing such cells, the method comprising removing from the sample the cell or cells that express PODXL on their surface.

10. The method of claim 9 wherein the sample is contacted with a binding moiety which moiety binds to PODXL and the said undifferentiated induced pluripotent stem cell(s) is removed from the sample by virtue of being bound to the binding moiety.

11. A method according to any one of Claims 1, 2 to 7 or 10 wherein the binding moiety is an antibody.

## Patentansprüche

1. Verfahren zum Trennen undifferenzierter induzierter pluripotenter Stammzellen von induzierten pluripotenten Stammzellen, die bereits eine Differenzierung durchlaufen haben oder diese gerade durchlaufen, wobei die undifferenzierten induzierten pluripotenten Stammzellen und induzierten pluripotenten Stammzellen, die bereits eine Differenzierung durchlaufen haben oder diese gerade durchlaufen, in einer Probe vorliegen, wobei das Verfahren Folgendes umfasst:
(i) das Kontaktieren der Probe mit einer Bindungsgruppierung, die in der Lage ist, Podocalyxin-ähnliches Protein (PODXL) zu binden, unter Bedingungen, die die Bindung der Gruppierung an auf der Oberfläche von Zellen in der Probe exprimiertes PODXL ermöglichen; und
(ii) das Trennen von an die Bindungsgruppierung gebundenen Zellen von nicht an die Gruppierung gebundenen Zellen.

2. Verfahren zur Anreicherung undifferenzierter induzierter pluripotenter Stammzellen aus einem Gemisch, das undifferenzierte induzierte pluripotente Stammzellen und induzierte pluripotente Stammzellen, die bereits eine Differenzierung durchlaufen haben oder diese gerade durchlaufen, umfasst, wobei das Verfahren Folgendes umfasst:
(i) das Kontaktieren des Gemischs mit einer Bindungsgruppierung, die in der Lage ist, Podocalyxin-ähnliches Protein (PODXL) zu binden, unter Bedingungen, die die Bindung der Gruppierung an auf der Oberfläche von Zellen in dem Gemisch exprimiertes PODXL ermöglichen; und
(ii) das Trennen von an die Gruppierung gebundenen Zellen von nicht an die Gruppierung gebundenen Zellen, um eine Zellpopulation zu erzeugen, die in Bezug auf undifferenzierte induzierte pluripotente Stammzellen angereichert ist.

3. Verfahren zur Anreicherung induzierter pluripotenter Stammzellen, die bereits eine Differenzierung durchlaufen haben oder diese gerade durchlaufen, aus einem Gemisch, das undifferenzierte induzierte pluripotente Stammzellen und induzierte pluripotente Stammzellen, die bereits eine Differenzierung durchlaufen haben oder diese gerade durchlaufen, umfasst, wobei das Verfahren Folgendes umfasst:
(i) das Kontaktieren des Gemischs mit einer Bindungsgruppierung, die in der Lage ist, Podocalyxin-ähnliches Protein (PODXL) zu binden, unter Bedingungen, die die Bindung der Gruppierung an auf der Oberfläche von Zellen in dem Gemisch exprimiertes PODXL ermöglichen; und
(ii) das Trennen von nicht an die Gruppierung gebundenen Zellen von an die Gruppierung gebundenen Zellen, um eine Zellpopulation zu erzeugen, die in Bezug auf induzierte pluripotente Stammzellen, die bereits eine Differenzierung durchlaufen haben oder diese gerade durchlaufen, angereichert ist.

4. Verfahren zur Herstellung einer Zusammensetzung, die aus undifferenzierten induzierten pluripotenten Stammzellen differenzierte Zellen enthält, wobei die Zusammensetzung im Wesentlichen keine undifferenzierten induzierten pluripotenten Stammzellen enthält, die PODXL auf ihrer Oberfläche exprimieren, wobei das Verfahren Folgendes umfasst:
(i) das Bereitstellen einer Population von Zellen, die undifferenzierte induzierte pluripotente Stammzellen und aus undifferenzierten induzierten pluripotenten Stammzellen differenzierte Zellen umfasst;
(ii) das Kontaktieren der Population mit einer Bindungsgruppierung, die in der Lage ist, Podocalyxin-ähnliches Protein (PODXL) zu binden, unter Bedingungen, die die Bindung der Gruppierung an auf der Oberfläche von Zellen in der Probe exprimiertes PODXL ermöglichen; und
(iii) das Trennen von nicht an die Bindungsgruppierung gebundenen Zellen von an die Gruppierung gebundenen Zellen.

5. Verfahren nach Anspruch 4, das außerdem das Mischen der nicht an die Bindungsgruppierung gebundenen abgetrennten Zellen mit einem pharmazeutisch annehmbaren Träger, Adjuvans oder Verdünnungsmittel umfasst.

6. Verfahren zur Herstellung einer Zusammensetzung, die undifferenzierte induzierte pluripotente Stammzellen enthält, die PODXL auf ihrer Oberfläche exprimieren, wobei die Zusammensetzung im Wesentlichen keine aus undifferenzierten induzierten pluripotenten Stammzellen differenzierten Zellen enthält, wobei das Verfahren Folgendes umfasst:
(i) das Bereitstellen einer Population von Zellen, die undifferenzierte induzierte pluripotente Stammzellen und aus undifferenzierten induzierten pluripotenten Stammzellen differenzierte Zellen umfasst;
(ii) das Kontaktieren der Population mit einer Bindungsgruppierung, die in der Lage ist, Podocalyxin-ähnliches Protein (PODXL) zu binden, unter Bedingungen, die die Bindung der Gruppierung an auf der Oberfläche von Zellen in der Probe exprimiertes PODXL ermöglichen; und
(iii) das Trennen von an die Bindungsgruppierung gebundenen Zellen von nicht an die Gruppierung gebundenen Zellen.

7. Verfahren nach Anspruch 6, das außerdem Folgendes umfasst:
(i) das Dissoziieren der Bindungsgruppierung von den abgetrennten Zellen, die an die Bindungsgruppierung gebunden sind, und/oder
(ii) das Mischen der abgetrennten Zellen, die an die Bindungsgruppierung gebunden waren, mit einem pharmazeutisch annehmbaren Träger, Adjuvans oder Verdünnungsmittel.

8. Verfahren zur Zerstörung einer undifferenzierten induzierten pluripotenten Stammzelle oder von undifferenzierten induzierten pluripotenten Stammzellen in einer Probe, die solche Zellen enthält, wobei das Verfahren das Zerstören der Zelle oder Zellen, die PODXL auf ihrer Oberfläche exprimiert/exprimieren, in der Probe umfasst.

9. Verfahren zur Entfernung einer undifferenzierten induzierten pluripotenten Stammzelle oder von undifferenzierten induzierten pluripotenten Stammzellen in einer Probe, die solche Zellen enthält, wobei das Verfahren das Entfernen der Zelle oder Zellen, die PODXL auf ihrer Oberfläche exprimiert/exprimieren, aus der Probe umfasst.

10. Verfahren nach Anspruch 9, wobei die Probe mit einer Bindungsgruppierung kontaktiert wird, die an PODXL bindet, und die undifferenzierte(n) induzierte(n) pluripotente(n) Stammzelle(n) aus der Probe dadurch entfernt wird/werden, indem sie an die Bindungsgruppierung gebunden wird/werden.

11. Verfahren nach einem der Ansprüche 1, 2 bis 7 oder 10, wobei die Bindungsgruppierung ein Antikörper ist.

## Revendications

1. Procédé pour séparer des cellules souches pluripotentes induites indifférenciées de cellules souches pluripotentes induites qui ont subi ou subissent une différenciation, dans lequel les cellules souches pluripotentes induites indifférenciées et les cellules souches pluripotentes induites qui ont subi ou subissent une différenciation sont présentes dans un échantillon, le procédé comprenant :
(i) la mise en contact de l'échantillon avec un fragment de liaison capable de se lier à une protéine de type podocalyxine (PODXL) dans des conditions permettant la liaison du fragment à une PODXL exprimée sur la surface des cellules dans l'échantillon ; et
(ii) une séparation entre les cellules liées au fragment de liaison et les cellules non liées au fragment.

2. Procédé d'enrichissement de cellules souches pluripotentes induites indifférenciées à partir d'un mélange comprenant des cellules souches pluripotentes induites indifférenciées et des cellules souches pluripotentes induites qui ont subi ou subissent une différenciation, le procédé comprenant :
(i) la mise en contact du mélange avec un fragment de liaison capable de se lier à une protéine de type podocalyxine (PODXL) dans des conditions permettant la liaison du fragment à une PODXL exprimée sur la surface des cellules dans le mélange ; et
(ii) une séparation entre les cellules liées au fragment et les cellules non liées au fragment de sorte à générer une population de cellules qui est enrichie en cellules souches pluripotentes induites indifférenciées.

3. Procédé d'enrichissement de cellules souches pluripotentes induites qui ont subi ou subissent une différenciation à partir d'un mélange comprenant des cellules souches pluripotentes induites indifférenciées et des cellules souches pluripotentes induites qui ont subi ou subissent une différenciation, le procédé comprenant :
(i) la mise en contact du mélange avec un fragment de liaison capable de se lier à une protéine de type podocalyxine (PODXL) dans des conditions permettant la liaison du fragment à une PODXL exprimée sur la surface des cellules dans le mélange ; et
(ii) une séparation entre les cellules non liées au fragment et les cellules liées au fragment de sorte à générer une population de cellules qui est enrichie en cellules souches pluripotentes induites qui ont subi ou subissent une différenciation.

4. Procédé de préparation d'une composition contenant des cellules différenciées à partir de cellules souches pluripotentes induites indifférenciées, laquelle composition ne contient essentiellement aucune cellule souche pluripotente induite indifférenciée qui exprime une PODXL sur sa surface, le procédé comprenant :
(i) la mise à disposition d'une population de cellules comprenant des cellules souches pluripotentes induites indifférenciées et des cellules différenciées à partir de cellules souches pluripotentes induites indifférenciées ;
(ii) la mise en contact de la population avec un fragment de liaison capable de se lier à une protéine de type podocalyxine (PODXL) dans des conditions permettant la liaison du fragment à une PODXL exprimée sur la surface des cellules dans l'échantillon ; et
(iii) une séparation entre les cellules non liées au fragment de liaison et les cellules liées au fragment.

5. Procédé selon la revendication 4, comprenant en outre le mélange des cellules séparées non liées au fragment de liaison avec un véhicule, adjuvant ou diluant pharmaceutiquement acceptable.

6. Procédé de préparation d'une composition contenant des cellules souches pluripotentes induites indifférenciées qui expriment une PODXL sur leur surface, laquelle composition ne contient essentiellement aucune cellule différenciée à partir des cellules souches pluripotentes induites indifférenciées, le procédé comprenant :
(i) la mise à disposition d'une population de cellules comprenant des cellules souches pluripotentes induites indifférenciées et des cellules différenciées à partir de cellules souches pluripotentes induites indifférenciées ;
(ii) la mise en contact de la population avec un fragment de liaison capable de se lier à une protéine de type podocalyxine (PODXL) dans des conditions permettant la liaison du fragment à une PODXL exprimée sur la surface des cellules dans l'échantillon ; et
(iii) une séparation entre les cellules liées au fragment de liaison et les cellules non liées au fragment.

7. Procédé selon la revendication 6, comprenant en outre :
(i) une dissociation entre le fragment de liaison et les cellules séparées liées au fragment de liaison, et/ou
(ii) le mélange des cellules séparées qui se sont liées au fragment de liaison avec un véhicule, adjuvant ou diluant pharmaceutiquement acceptable.

8. Procédé de destruction d'une cellule ou de cellules souche(s) pluripotente(s) induite(s) indifférenciée (s) dans un échantillon contenant de telles cellules, le procédé comprenant la destruction de la cellule ou des cellules dans l'échantillon qui exprime/expriment une PODXL sur sa/leur surface.

9. Procédé d'élimination d'une cellule ou de cellules souche(s) pluripotente(s) induite(s) indifférenciée(s) à partir d'un échantillon contenant de telles cellules, le procédé comprenant l'élimination, à partir de l'échantillon, de la cellule ou des cellules qui exprime/expriment une PODXL sur sa/leur surface.

10. Procédé selon la revendication 9, dans lequel l'échantillon est mis en contact avec un fragment de liaison, lequel fragment se lie à une PODXL et ladite/lesdites cellule(s) souche(s) pluripotente(s) induite(s) indifférenciée(s) est/sont éliminée(s) de l'échantillon du fait d'être liée(s) au fragment de liaison.

11. Procédé selon l'une quelconque des revendications 1, 2 à 7 ou 10, dans lequel le fragment de liaison est un anticorps.
